# EUROPEAN PATENT APPLICATION

(11) **EP 4 458 345 A1**
(43) Date of publication of application: **06.11.2024**
(21) Application number: 23382413.5
(22) Date of filing: 04.05.2023
(51) Int. Cl.: A61K 8/06, A61K 8/22, A61K 8/41, A61K 8/92, A61K 8/64, A61K 8/34, A61K 8/86, A61Q 5/10

(54) **COMPOSITIONS FOR DYEING KERATIN FIBERS**

(71) Applicant: Beautyge, S.L., 08940 Cornella de Llobregat (ES)
(72) Inventor: DÍAZ POZO, Óscar, 08940 Cornella de Llobregat (ES); MURO URANGA, Ander, 08940 Cornella de Llobregat (ES); VALLECILLOS LÓPEZ, Rocío, 08940 Cornella de Llobregat (ES)
(74) Representative: Gallardo, Antonio M.

(57) **Abstract**

Compositions for dyeing keratin fibers are described. Said dyeing compositions comprise, in a cosmetically acceptable medium: (a) at least one hydrolyzed protein or peptide or amino acid, and mixtures thereof, (b) hydrolyzed jojoba esters; (c) at least one alkanolamine, a C₁-C₄ alkyl alkanolamine, and mixtures thereof; (d) at least one oxidation dye, and (e) at least one oxidizing agent.

These dyeing compositions cause reduced hair damage and, at the same time, present a faster color processing and improved color retention (i.e. slower rate of color loss).

A process for dyeing keratin fibers using these compositions and a packaging kit containing these compositions are also described.

## Description

### TECHNICAL FIELD

Compositions for dyeing keratin fibers are described. Said dyeing compositions comprise, in a cosmetically acceptable medium, based on the total weight of the composition: (a) at least one hydrolyzed protein or peptide or amino acid, and mixtures thereof, (b) hydrolyzed jojoba esters; (c) at least one alkanolamine, a C₁-C₄ alkyl alkanolamine, and mixtures thereof; (d) at least one oxidation dye, and (e) at least one oxidizing agent. These dyeing compositions cause reduced hair damage and, at the same time, present a faster color processing and improved color retention (i.e. slower rate of color loss).

### BACKGROUND

The information provided below is not admitted to be prior art to the present disclosure, but is provided solely to assist the understanding of the reader.

It is known for centuries to dye keratinous fibers, for example human hair, with dyeing compositions comprising dyes or dye precursors.

Most hair dyeing products fall under four major groupings: temporary, semi-permanent, demi-permanent, and permanent.

Temporary hair dyeing is usually a leave on product that causes minimal damage to the hair. However, temporary hair dyeing causes stains, and leaches out under rain or with perspiration. Temporary hair dyeing washes out with the next shampoo.

Semi-permanent hair dyeing comes as a rinse, and it causes minimal damage to the hair. However, semi-permanent hair dyeing washes out to some degree with each shampoo and washes out completely within about 4 to 6 shampoos.

Demi-permanent hair dyeing last longer that semi-permanent hair dyeing after repeated washing and exposure to sunlight. Both methods are temporary compared to permanent hair color, with demi- lasting up to 30 shampoos.

Demi-permanent contains no ammonia but includes a low-volume developer, containing an oxidizing agent such as, for example, hydrogen peroxide, which allows the dye to penetrate under the outer cuticle of the hair and, in addition, damaging the hair to some extend.

Permanent hair dyeing is characterized by excellent, long-lasting color results. It generally comes in two parts: a dye solution and a developer solution, which contains an oxidizing agent such as, for example, hydrogen peroxide. Usually, permanent hair dyeing compositions do not contain dyestuffs in the conventional sense of the word. They contain colorless precursors which will react with the oxidizing agent inside the hair fiber to produce colored molecules. The dye solution (tint solution) and the developer solution are mixed and then applied to the hair, which is then left for about 10 to about 50 minutes and then rinsed with water.

The oxidizing agent (e.g., hydrogen peroxide) initiates not only the formation of the dyes, but it also breaks down oxidatively the hair's own color pigments (melanins), so a lightening coloring is also possible simultaneously. In order to produce satisfactory coloring and lightening, permanent hair dyeing usually requires an alkaline pH during use; optimal results are achieved in particular at pH values between about 8 and 12.

Ammonia is the alkalizing agent of choice today for adjusting these pH values. Not only can the pH range necessary for dye formation be adjusted with ammonia, but ammonia also causes the swelling of hair to a greater degree than all other known alkalizing agents. At the same time, ammonia acts as a penetration agent or penetration aid, also to a greater extent than all other commercial alkalizing agents.

For these reasons, more intensive colors and significantly better gray coverage are obtained during use of ammonia in permanent hair coloration in comparison with other alkalizing agents (such as, for example, potassium or sodium hydroxide, alkanolamines, or carbonates such as sodium carbonate or potassium carbonate).

The performance advantages associated with the use of ammonia are so diverse that ammonia is used in a great number of permanent hair coloration despite its unpleasant, pungent odor.

However, it would be desirable to provide the consumer with compositions and methods that can treat the hair, e.g. lift the color of hair and/or deposit color onto hair in an effective manner, while providing other cosmetic advantages such as shine conditioning, fiber strength and/or healthy appearing but avoiding or minimizing damage to the hair.

Accordingly, there is a need for compositions for dyeing keratin fibers with a reduced hair damage and, at the same time, with a faster color processing and an improved color retention (i.e. slower rate of color loss).

### SUMMARY

Described herein is a composition for dyeing keratin fibers comprising, in a cosmetically acceptable medium:
(a) at least one hydrolyzed protein or peptide or amino acid, and mixtures thereof,
(b) hydrolyzed jojoba esters;
(c) at least one alkanolamine, a C₁-C₄ alkyl alkanolamine, and mixtures thereof;
(d) at least one oxidation dye, and
(e) at least one oxidizing agent.

In an embodiment disclosed herein the at least one hydrolyzed protein is selected from the group consisting of hydrolyzed silk protein, hydrolyzed pea protein, hydrolyzed soy protein, hydrolyzed wheat protein, hydrolyzed corn protein, and mixtures thereof.

In an embodiment disclosed herein the at least at least one alkanolamine or C₁-C₄ alkyl alkanolamine is selected from the group consisting of monoethanolamine (MEA), 2-amino-1-propanol (2A1P), 2-amino-1-butanol (2A1B), 2-amino-2-methyl-1-propanol (AMP), 2-dimethylamino-2-methyl-1-propanol (DMAMP), tris(hydroxymethyl)aminomethane (tromethamine), N,N-diethylethanolamine (DEEA), N,N-dimethylethanolamine (DMEA), N-methyldiethanolamine (MDEA), N-methylethanolamine (NMEA), and mixtures thereof.

In an embodiment disclosed herein the at least one alkanolamine or C₁-C₄ alkyl alkanolamine is selected from the group consisting of monoethanolamine (MEA), 2-amino-2-methyl-1-propanol (AMP), and mixtures thereof.

In an embodiment disclosed herein the at least one oxidizing agent is hydrogen peroxide.

In an embodiment disclosed herein the total amount of (a) the at least one hydrolyzed protein or peptide or amino acid, and mixtures thereof, is in the range of about 0.05 to 10 wt.%, based on the total weight of the composition.

In an embodiment disclosed herein the total amount of (b) hydrolyzed jojoba esters is the range of about 0.05 to 10 wt.%, based on the total weight of the composition.

In an embodiment disclosed herein the total amount of (c) the at least one alkanolamine, a C₁-C₄ alkyl alkanolamine, and mixtures thereof, is in the range of about 0.05 to 10 wt.%, based on the total weight of the composition.

In an embodiment disclosed herein the dyeing composition comprises, in a cosmetically acceptable medium, based on the total weight of the composition:
(a) about 0.05-10 wt.% of at least one hydrolyzed protein or peptide or amino acid, and mixtures thereof,
(b) about 0.05-10 wt.% of hydrolyzed jojoba esters;
(c) about 0.05-10 wt.% of at least one alkanolamine, a C₁-C₄ alkyl alkanolamine, and mixtures thereof;
(d) about 0.001-10 wt.% of at least one oxidation dye, and
(e) about 0.05-10 wt.% of at least one oxidizing agent.

In an embodiment disclosed herein the dyeing composition comprises, in a cosmetically acceptable medium, based on the total weight of the composition:
(a) about 0.05-10 wt.% of at least one hydrolyzed protein or peptide or amino acid, or mixtures thereof, selected from the group consisting of hydrolyzed silk protein, hydrolyzed pea protein, hydrolyzed soy protein, hydrolyzed wheat protein, hydrolyzed corn protein, and mixtures thereof;
(b) about 0.05-10 wt.% of hydrolyzed jojoba esters;
(c) about 0.05-10 wt.% of at least one alkanolamine, a C₁-C₄ alkyl alkanolamine, or mixtures thereof, selected from the group consisting of monoethanolamine (MEA), 2-amino-1-propanol (2A1P), 2-amino-1-butanol (2A1B), 2-amino-2-methyl-1-propanol (AMP), 2-dimethylamino-2-methyl-1-propanol (DMAMP), tris(hydroxymethyl)aminomethane (tromethamine), N,N-diethylethanolamine (DEEA), N,N-dimethylethanolamine (DMEA), N-methyldiethanolamine (MDEA), N-methylethanolamine (NMEA), and mixtures thereof;
(d) about 0.001-10 wt.% of at least one oxidation dye, and
(e) about 0.05-10 wt.% of at least one oxidizing agent.

In an embodiment disclosed herein the keratin fibers are human hair.

In an embodiment disclosed herein the dyeing composition has a pH in the range from 5.5 to 8.0.

An embodiment disclosed herein is a process for dyeing keratin fibers comprising the steps of:
i) applying a dyeing composition as defined above to the keratin fibers for a period which is sufficient to obtain the dyeing effect;
ii) removing the dyeing composition from the keratin fibers by rinsing with water.

In embodiment disclosed herein, the process for dyeing keratin fibers is intended for reducing the damage of the keratin fibers.

An embodiment disclosed herein is a multi-compartment device for dyeing keratin fibers comprising at least two compartments packaged separate from one another, wherein
(i) one compartment comprises a composition for dyeing keratin fibers comprising:
   (a) at least one hydrolyzed protein or peptide or amino acid, and mixtures thereof,
   (b) hydrolyzed jojoba esters;
   (c) at least one alkanolamine, a C₁-C₄ alkyl alkanolamine, and mixtures thereof;
   (d) at least one oxidation dye, and
(ii) a second compartment comprises a hydrogen peroxide composition.

### DETAILED DESCRIPTION

Embodiments of the present disclosure are discussed in detail below. In describing embodiments, specific terminology is employed for the sake of clarity. However, the disclosure is not intended to be limited to the specific terminology so selected. While specific exemplary embodiments are discussed, it should be understood that this is done for illustration purposes only. A person skilled in the relevant art will recognize that other components and configurations can be used without parting from the spirit and scope of the disclosure. While a number of embodiments and features are described herein, it is to be understood that the various features of the disclosure and aspects of embodiments, even if described separately, may be combined unless mutually exclusive or contrary to the specific description. All references cited herein are incorporated by reference as if each had been individually incorporated.

The terms "about" or "approximately" as used herein shall generally mean within 10 percent of a given value. For example, the weight proportion of a compound of about 1% means a weight proportion ranging from 0.9 to 1.1.

Other than in the operating examples, or where otherwise indicated, all numbers expressing quantities of ingredients and/or reaction conditions are to be understood as being modified in all instances by the term "about".

The ranges defined in the specification include the end values as well, i.e. a range of 1 to 10 implies that both 1 and 10 are included in the range. For the avoidance of doubt, the applicant shall be entitled to any equivalents according to applicable law.

The term "keratin fibers" as used herein refers to a family of proteins that occur in the vertebrates and exercise a protective function. In an embodiment, keratin fibers refers to human keratin fibers. In another embodiment, keratin fibers refers to human hair.

The term "comprising" refers to optional compatible components/steps that can be used provided that the important ingredients/steps are present.

The term "comprising" thus encompasses and includes the more restrictive terms "consisting of" and "consisting essentially of".

The term "alkanolamines" as used herein refers to chemical compounds that contain both hydroxyl (-OH) and amino functional groups on an alkane backbone.

The term "alkyl alkanolamines" as used herein refers chemical compounds that contain a basic secondary or tertiary nitrogen atom and at least one hydroxyl group.

The term "hydrolyzed jojoba esters," as used herein, refers to the mixture obtained by the hydrolysis of jojoba esters by acid, enzyme or other methods of hydrolysis.

The term "hydrolyzed protein," as used herein, refers to the product of the hydrolysis of homogeneous or heterogeneous proteins, or their respective components, derivatives or combinations thereof, from sources including, but not limited to, plants and their respective components, seeds, animal bones, connective tissue, animal keratin, bovine and porcine collagen, human hair, wool, silk, elastin, reticulin, milk, egg, wheat, corn, soy, oats, casein, albumin, or any collagenous or keratin substance, or derivatives thereof.

The term "peptide" refers to two or more amino acids joined to each other by peptide bonds or modified peptide bonds.

The term "amino acid" refers to the basic chemical structural unit of a protein or a peptide.

The term "oxidation dye " refers to typically colorless materials which will react with an oxidizing agent (typically hydrogen peroxide) inside the hair fiber to produce colored molecules. Oxidation dyes are categorized under two groups: precursors (bases) and couplers. The precursors and peroxide diffuse into the hair shaft, where color formation takes place after a cascade of chemical reactions. The dye precursors are oxidized by hydrogen peroxide to p-benzoquinone imines/diimines, which are reactive intermediates in the color formation. The couplers, which are relatively stable to hydrogen peroxide, undergo rapid reaction with the intermediates resulting in dinuclear, trinuclear or polynuclear colorant molecules. These molecules are too large to escape from the hair structure.

The term "oxidizing agent" refers to an electron accepting compound suitable for use in hair dyeing compositions for removing the natural color of hair (by destroying the melanin pigment) and reacting with oxidative primary dye precursors to enable the reaction with the oxidative coupler dye precursors to form the color dye products. The most commonly used oxidizing agent in the art is hydrogen peroxide, however further suitable oxidizing agents that can be used in combination with hydrogen peroxide are described below. Oxidizing agents also include those which produce hydrogen peroxide when contacted with water. For example sodium percarbonate, sodium perborate and urea peroxide can be used as anhydrous materials, which when contacted with water release hydrogen peroxide.

The term "INCI" is the acronym of International Nomenclature Cosmetic Ingredient. INCI names are systematic names internationally recognized to identify cosmetic ingredients. They are developed by the International Nomenclature Committee (INC) and published by the Personal Care Products Council (PCPC) in the *International Cosmetic Ingredient Dictionary and Handbook.*

Typically, the composition for dyeing keratin fibers comprises, in a cosmetically acceptable medium:
(a) at least one hydrolyzed protein or peptide or amino acid, and mixtures thereof,
(b) hydrolyzed jojoba esters;
(c) at least one alkanolamine, a C₁-C₄ alkyl alkanolamine, and mixtures thereof;
(d) at least one oxidation dye, and
(e) at least one oxidizing agent.

### Cosmetically acceptable medium

The cosmetically acceptable medium for the dyeing composition is typically an aqueous medium consisting of water and may advantageously contain cosmetically acceptable organic solvents including more particularly alcohols such as ethyl alcohol, isopropyl alcohol, benzyl alcohol and phenylethyl alcohol, glycols or glycol ethers such as, for example, monomethyl, monoethyl and monobutyl ethers of ethylene glycol, propylene glycol or its ethers such as, for example, monomethyl ether of propylene glycol, butylene glycol, dipropylene glycol as well as the alkyl ethers of diethylene glycol such as for example monoethyl ether or monobutyl ether of diethylene glycol. Water may be present in the range of about 30 to about 90 wt. %, or about 40 to about 85 wt. %, or about 45 to about 80 wt.%, based on the total weight of the composition. The organic solvents may then be present in concentrations of between about 0.5 and 20 wt. %, or about 2 and 15 wt.%, based on the total weight of the composition.

### The hydrolyzed protein or peptide or amino acid

As indicated above, the composition for dyeing keratin fibers may comprise (a) at least one hydrolyzed protein or peptide or amino acid, and mixtures thereof.

The term "hydrolyzed protein" includes hydrolyzed proteins produced from naturally occurring proteins. Synthetic proteins, peptides, or amino acids as well as naturally occurring proteins, peptides or amino acids or mixtures of naturally occurring and synthetic proteins and/or peptides and/or amino acids may also be used. Hydrolyzed protein prepared from various proteins, their respective components, and derivatives may be combined and used in the composition of the present disclosure. Moreover, a hydrolyzed protein may be supplemented by the addition of one or more natural or synthetic peptides or amino acids.

Methods for producing hydrolyzed proteins from the abovementioned protein sources include, but are not limited to: 1) acid hydrolysis; 2) alkali hydrolysis; and 3) enzyme hydrolysis using a suitable protease.

These methods, along with several others, for preparing hydrolyzed proteins are well known in the art. Further, hydrolyzed proteins suitable for the compositions of the present disclosure are commercially available.

Hydrolyzed proteins may have a number average molecular weight from about two hundred to several hundred thousand depending on the nature of the protein and/or the extent of hydrolysis. The number average molecular weight of the hydrolyzed proteins suitable for the present disclosure is from about 100 to 100,000, or from about 100 to 20,000, or from about 100 to 10,000, or from about 100 to 5,000.

In an embodiment, the hydrolyzed protein is selected from the group consisting of hydrolyzed silk protein, hydrolyzed pea protein, hydrolyzed soy protein, hydrolyzed wheat protein, hydrolyzed corn protein, and mixtures thereof, particularly from hydrolyzed silk protein, hydrolyzed pea protein, hydrolyzed soy protein, hydrolyzed wheat protein, hydrolyzed corn protein, and mixtures thereof.

In another embodiment, the hydrolyzed protein is selected from the group consisting of hydrolyzed pea protein, hydrolyzed soy protein, hydrolyzed wheat protein, hydrolyzed corn protein, and mixtures thereof, particularly from hydrolyzed pea protein and a mixture comprising hydrolyzed soy protein, hydrolyzed wheat protein and hydrolyzed corn protein, and mixtures thereof.

In a particular embodiment, the hydrolyzed protein is selected from the group consisting of a mixture comprising hydrolyzed soy protein, hydrolyzed wheat protein and hydrolyzed corn protein.

Examples of suitable amino acids include the twenty amino acids that form the proteins of living organisms (standard proteinogenic amino acids): alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, and valine. The amino acids of the present disclosure, however, are not limited to the standard proteinogenic amino acids.

Non-limiting examples of amino sulfonic acids include aminomethane sulfonic acid, 2-aminoethane sulfonic acid (taurine), aminopropane sulfonic acid, aminobutane sulfonic acid, aminohexane sulfonic acid, aminoisopropyl sulfonic acid, aminododecyl sulfonic acid, aminobenzene sulfonic acid, aminotoulene sulfonic acid, sulfanilic acid, chlorosulfanilic acid, diamino benzene sulfonic acid, amino phenol sulfonic acid, amino propyl benzene sulfonic acid, amino hexyl benzene sulfonic acid, and mixtures thereof.

In an embodiment, the at least one amino acid is selected from the group consisting of glycine, alanine, serine, beta-alanine, taurine, sodium glycinate, sodium alaninate, sodium serinate, lithium beta-alanine, sodium taurate, and mixtures thereof.

In a particular embodiment, the at least one hydrolyzed protein or peptide or amino acid (a) is selected from the group consisting of hydrolyzed pea protein, hydrolyzed soy protein, hydrolyzed wheat protein, hydrolyzed corn protein, glycine, alanine, serine, beta-alanine, taurine, sodium glycinate, sodium alaninate, sodium serinate, lithium beta-alanine, sodium taurate, and mixtures thereof.

In another particular embodiment, the at least one hydrolyzed protein or peptide or amino acid (a) is selected from the group consisting of hydrolyzed pea protein, hydrolyzed soy protein, hydrolyzed wheat protein, hydrolyzed corn protein, glycine, taurine, and mixtures thereof.

In another particular embodiment, the at least one hydrolyzed protein or peptide or amino acid (a) is selected from the group consisting of hydrolyzed pea protein, a mixture comprising hydrolyzed soy protein, hydrolyzed wheat protein and hydrolyzed corn protein, glycine, taurine and mixtures thereof.

In another particular embodiment, the at least one hydrolyzed protein or peptide or amino acid (a) is selected from the group consisting of a mixture comprising hydrolyzed soy protein, hydrolyzed wheat protein and hydrolyzed corn protein.

Suitable hydrolyzed proteins for use in the present disclosure include, but are not limited to,
- CROPEPTIDE (INCI: Hydrolyzed Wheat Protein and Hydrolyzed Wheat Starch), CROSILK 10000 (INCI: Hydrolyzed Silk Protein), HYDROLACTIN 2500 (INCI: Hydrolyzed Milk Protein), HYDROSOLANUM (INCI: Hydrolyzed Vegetable Protein), HYDROSOY 2000 PE (INCI: Hydrolyzed Soy Protein), HYDROTRITICUM 2000 PE (INCI: Hydrolyzed Wheat Protein), or HYDROSATIVUM P (INCI: Hydrolyzed Pea Protein), all available from Croda;
- MILK-TEIN (INCI: Hydrolyzed Milk Protein), Rice PRO-TEIN (INCI: Hydrolyzed Rice Protein), FLAX-TEIN Pro (hydrolyzed linseed protein; Tri-K), SOY-TEIN NL (INCI: Hydrolyzed Soy Protein), WHEAT-TEIN W (INCI: Hydrolyzed Wheat Protein; Tri-K), or KERAPLANT Z NPNF (INCI: Hydrolyzed Wheat Protein (and) Hydrolyzed Soy Protein (and) Hydrolyzed Corn Protein), all available from Tri-K.

Particularly suitable hydrolyzed proteins for use in the present disclosure are HYDROSATIVUM P (INCI: Hydrolyzed Pea Protein), available from Croda in the form of a clear yellow-amber aqueous solution containing about 20% of active material; or KERAPLANT Z NPNF (INCI: Hydrolyzed Wheat Protein (and) Hydrolyzed Soy Protein (and) Hydrolyzed Corn Protein), from Tri-K, in the form of an aqueous solution containing 7.0-14.0% of Hydrolyzed Wheat Protein, 3.0-8.0% of Hydrolyzed Soy Protein and 3.0-8.0% of Hydrolyzed Corn Protein.

In the composition for dyeing keratin fibers, the total amount of (a) the at least one hydrolyzed protein or peptide or amino acid, and mixtures thereof, may be in the range of about 0.05 to 10 wt.%, or about 0.1 to about 5 wt.%, or about 0.15 to 3 wt.% or about 0.2 to about 2 wt.%, based on the total weight of the composition.

### Hydrolyzed jojoba esters

Hydrolyzed jojoba esters are derived from jojoba oil. Jojoba oil (INCI: Simmondsia Chinensis (Jojoba) Seed Oil) primarily consists of straight chain monoesters of C₂₀ and C₂₂ alcohols and fatty acids with one point of unsaturation on each side of the ester linkage.

According to the Personal Care Products Council ("PCPC") On-Line INFOBASE (resource providing access to personal care and cosmetic collections on ingredients, technical reports, and current news), hydrolyzed jojoba esters is the hydrolysate of Jojaba Esters derived by acid, enzyme or other methods of hydrolysis.

Suitable hydrolyzed jojoba esters for use in the present disclosure are available under the trademark Floraesters K-20W Jojoba (INCI: Hydrolyzed Jojoba Esters (and) Water (aqua)) and Floraesters K-100W Jojoba (INCI: Hydrolyzed Jojoba Esters (and) Jojoba Esters (and) Water (aqua)), both supplied by the company International Flora Technologies, Ltd. (Floratech).

Floraesters K-20W Jojoba is comprised of 20% hydrolyzed jojoba esters (i.e. potassium jojobate and jojoba alcohols) and 80% water, while Floraesters K-100 Jojoba comprised of 80% hydrolyzed jojoba esters, 10% residual jojoba esters, and 10% water.

In an embodiment, the hydrolyzed jojoba esters comprise potassium jojobate and jojoba alcohol.

According to the Personal Care Products Council ("PCPC") On-Line INFOBASE Potassium Jojobate is the potassium salt of the acids derived from Simmondsia Chinensis (Jojoba) Seed Oil.

According to the Personal Care Products Council ("PCPC") On-Line INFOBASE Jojoba Alcohol is the alcohol fraction obtained by the saponification of Simmondsia Chinensis (Jojoba) Seed Oil.

In the composition for dyeing keratin fibers, the total amount of (b) hydrolyzed jojoba esters, may be in the range of about 0.05 to 10 wt.%, or about 0.1 to about 5 wt.%, or about 0.15 to 3 wt.% or about 0.2 to about 2 wt.%, based on the total weight of the composition.

### The alkanolamines and the C₁-C₄ alkyl alkanolamines

As indicated above, the composition for dyeing keratin fibers may comprise (c) at least one alkanolamine, a C₁-C₄ alkyl alkanolamine, and mixtures thereof.

Typically, the alkanolamines and the C₁-C₄ alkyl alkanolamines are selected from the group consisting of monoethanolamine (MEA), diethanolamine, triethanolamine, isopropanolamine, 2-amino-1-propanol (2A1P), 2-amino-1-butanol (2A1B), 2-amino-2-methyl-1-propanol (AMP), 2-dimethylamino-2-methyl-1-propanol (DMAMP), tris(hydroxymethyl)aminomethane (tromethamine), N,N-diethylethanolamine (DEEA), N,N-dimethylethanolamine (DMEA), N-methyldiethanolamine (MDEA), N-methylethanolamine (NMEA), and mixtures thereof.

In an embodiment, the at least one alkanolamine or C₁-C₄ alkyl alkanolamine is selected from the group consisting of monoethanolamine (MEA), 2-amino-1-propanol (2A1P), 2-amino-1-butanol (2A1B), 2-amino-2-methyl-1-propanol (AMP), 2-dimethylamino-2-methyl-1-propanol (DMAMP), tris(hydroxymethyl)aminomethane (tromethamine), N,N-diethylethanolamine (DEEA), N,N-dimethylethanolamine (DMEA), N-methyldiethanolamine (MDEA), N-methylethanolamine (NMEA), and mixtures thereof.

In a particular embodiment, the at least one alkanolamine or C₁-C₄ alkyl alkanolamine is selected from the group consisting of monoethanolamine (MEA), 2-amino-1-propanol (2A1P), 2-amino-1-butanol (2A1B), 2-amino-2-methyl-1-propanol (AMP), and mixtures thereof.

In another particular embodiment, the at least one alkanolamine is monoethanolamine (MEA).

In the composition for dyeing keratin fibers, the total amount of (c) the at least one alkanolamine, a C₁-C₄ alkyl alkanolamine, and mixtures thereof, may be in the range of about 0.05 to 10 wt.%, or about 0.1 to about 5 wt.%, or about 0.15 to 3 wt.% or about 0.2 to about 2 wt.%, or about 0.3 to 1.5 wt.% based on the total weight of the composition.

### The oxidation dye

As indicated above, the composition for dyeing keratin fibers may comprise (d) at least one oxidation dye. The oxidation dyes are generally chosen from one or more oxidation precursors (i.e. bases) (d1) optionally combined with one or more couplers (d2). Examples of suitable oxidation precursors (d1) include, but are not limited to, p-phenylenediamine, p-toluenediamine, 2-(2-hydroxyethyl)-p-phenylenediamine, 2-(1,2-dihydroxyethyl)-p-phenylenediamine, N,N-bis(2-hydroxyethyl)-p-phenylenediamine, 2-methoxymethyl-p-phenylenediamine, N-(4-amino-3-methylphenyl)-N-[3-(1H-imidazol-1-yl)propyl]amine, N,N'-bis(2-hydroxyethyl)-N,N'-bis(4-aminophenyl)-1,3-diaminopropan-2-ol, bis(2-hydroxy-5-aminophenyl)methane, 1,3-bis(2,5-diaminophenoxy)propan-2-ol, N,N'-bis(4-aminophenyl)-1,4-diazacycloheptane, 1,10-bis(2,5-diaminophenyl)-1,4,7,10-tetraoxadecane, p-aminophenol, 4-amino-3-methylphenol, 4-amino-2-aminomethylphenol, 4-amino-2-(1,2-dihydroxyethyl)phenol, 4-amino-2-(diethylaminomethyl)phenol, 4,5-diamino-1-(2-hydroxyethyl)-1H-pyrazole, 2,4,5,6-tetraaminopyrimidine, 4-hydroxy-2,5,6-triaminopyrimidine, 2-hydroxy-4,5,6-triaminopyrimidine, 2,3-diamino-6,7-dihydro-1H,5H-pyrazolo[1,2-a]pyrazol-1-one, and the physiologically acceptable salts thereof.

Examples of suitable couplers (d2) include, but are not limited to, 3-aminophenol, 5-amino-2-methylphenol, 3-amino-2-chloro-6-methylphenol, 2-hydroxy-4-aminophenoxyethanol, 5-amino-4-chloro-2-methylphenol, 5-(2-hydroxyethyl)amino-2-methylphenol, 2,4-dichloro-3-aminophenol, 2-aminophenol, 3-phenylenediamine, 2-(2,4-diaminophenoxy)ethanol, 1,3-bis(2,4-diaminophenoxy)propane, 1-methoxy-2-amino-4-(2-hydroxyethylamino)benzene, 1,3-bis(2,4-diaminophenyl)propane, 2,6-bis(2'-hydroxyethylamino)-1-methylbenzene, 2-({3-[(2-hydroxyethyl)amino]-4-methoxy-5-methylphenyl}amino)ethanol, 2-({3-[(2-hydroxyethyl)amino]-2-methoxy-5-methylphenyl}-amino)ethanol, 2-({3-[(2-hydroxyethyl)amino]-4,5-dimethylphenyl}amino)ethanol, 2-[3-morpholin-4-ylphenyl)amino]ethanol, 3-amino-4-(2-methoxyethoxy)-5-methylphenylamine, 1-amino-3-bis(2-hydroxyethyl)aminobenzene, resorcinol, 2-methylresorcinol, 4-chlororesorcinol, 1,2,4-trihydroxybenzene, 2-amino-3-hydroxypyridine, 3-amino-2-methylamino-6-methoxypyridine, 2,6-dihydroxy-3,4-dimethylpyridine, 3,5-diamino-2,6-dimethoxypyridine, 1-phenyl-3-methylpyrazol-5-one, 1-naphthol, 1,5-dihydroxynaphthalene, 2,7-dihydroxynaphthalene, 1,7-dihydroxynaphthalene, 1,8-dihydroxynaphthalene, 4-hydroxyindole, 6-hydroxyindole, 7-hydroxyindole, 4-hydroxyindoline, 6-hydroxyindoline, 7-hydroxyindoline, or mixtures of said compounds or the physiologically acceptable salts thereof

Typically, in the composition for dyeing keratin fibers, the total amount of (d) oxidation dyes (including oxidation precursors (d1) and couplers (d2)) is in the range of about 0.001 to about 10 wt.%, or about 0.01 to about 8.0 wt. %, or about 0.1 to about 7.0 wt.%, or about 0.2 to about 6.0 wt.%, based on the total weight of the composition.

### The oxidizing agent

Typically, the oxidizing agents are water-soluble inorganic peroxide materials which are capable of yielding hydrogen peroxide in an aqueous solution.

In an embodiment, the oxidizing agent (e) is selected from the group consisting of hydrogen peroxide, inorganic alkali metal peroxides (such as sodium periodate and sodium peroxide), organic peroxides (such as urea peroxide, melamine peroxide), inorganic perhydrate salt bleaching compounds (such as the alkali metal salts of perborates, percarbonates, perphosphates, persilicates, persulphates), and mixtures thereof; preferably from the group consisting of hydrogen peroxide, persulphates, and mixtures thereof.

In a particular embodiment, the oxidizing agent (e) is hydrogen peroxide.

The term "water-soluble" as used herein means that at normal temperature and pressure (NTP), defined as 20°C of temperature and 1 atm (101.325 kPa) of pressure, at least 0.1 g, 1 g, or 10 g of the oxidizing agent can be dissolved in 1 L (liter) of deionized water.

In the composition for dyeing keratin fibers, the total amount of (e) the oxidizing agent, may be in the range of about 0.5 to 8.0 wt.%, or about 0.6 to 6.0 wt.%, or about 0.65 to 5 wt.% or about 0.7 to about 3 wt.%, or about 0.75 to less than 1 wt.%, based on the total weight of the composition.

The oxidizing agent can be provided in aqueous solution or as a powder which is dissolved prior to use.

### The compositions for dyeing keratin fibers

In one embodiment, the composition for dyeing keratin fibers comprises, in a cosmetically acceptable medium:
(a) at least one hydrolyzed protein or peptide or amino acid, and mixtures thereof,
(b) hydrolyzed jojoba esters;
(c) at least one alkanolamine, a C₁-C₄ alkyl alkanolamine, and mixtures thereof;
(d) at least one oxidation dye, and
(e) at least one oxidizing agent.

In an embodiment, the composition for dyeing keratin fibers comprises, in a cosmetically acceptable medium:
(a) at least one hydrolyzed protein or peptide or amino acid, selected from the group consisting of hydrolyzed silk protein, hydrolyzed pea protein, hydrolyzed soy protein, hydrolyzed wheat protein, hydrolyzed corn protein, and mixtures thereof;
(b) hydrolyzed jojoba esters;
(c) at least one alkanolamine or a C₁-C₄ alkyl alkanolamine, selected from the group consisting of monoethanolamine (MEA), diethanolamine, triethanolamine, isopropanolamine, 2-amino-1-propanol (2A1P), 2-amino-1-butanol (2A1B), 2-amino-2-methyl-1-propanol (AMP), 2-dimethylamino-2-methyl-1-propanol (DMAMP), tris(hydroxymethyl)aminomethane (tromethamine), N,N-diethylethanolamine (DEEA), N,N-dimethylethanolamine (DMEA), N-methyldiethanolamine (MDEA), N-methylethanolamine (NMEA), and mixtures thereof;
(d) at least one oxidation dye, and
(e) at least one oxidizing agent.

In an embodiment, the composition for dyeing keratin fibers comprises, in a cosmetically acceptable medium:
(a) at least one hydrolyzed protein or peptide or amino acid, selected from the group consisting of hydrolyzed pea protein, hydrolyzed soy protein, hydrolyzed wheat protein, hydrolyzed corn protein, glycine, alanine, serine, beta-alanine, taurine, sodium glycinate, sodium alaninate, sodium serinate, lithium beta-alanine, sodium taurate, and mixtures thereof;
(b) hydrolyzed jojoba esters;
(c) at least one alkanolamine or a C₁-C₄ alkyl alkanolamine, selected from the group consisting of monoethanolamine (MEA), diethanolamine, triethanolamine, isopropanolamine, 2-amino-1-propanol (2A1P), 2-amino-1-butanol (2A1B), 2-amino-2-methyl-1-propanol (AMP), 2-dimethylamino-2-methyl-1-propanol (DMAMP), tris(hydroxymethyl)aminomethane (tromethamine), N,N-diethylethanolamine (DEEA), N,N-dimethylethanolamine (DMEA), N-methyldiethanolamine (MDEA), N-methylethanolamine (NMEA), and mixtures thereof;
(d) at least one oxidation dye, and
(e) at least one oxidizing agent.

In another embodiment, the composition for dyeing keratin fibers comprises, in a cosmetically acceptable medium:
(a) at least one hydrolyzed protein or peptide or amino acid, selected from the group consisting of hydrolyzed pea protein, hydrolyzed soy protein, hydrolyzed wheat protein, hydrolyzed corn protein, glycine, taurine, and mixtures thereof;
(b) hydrolyzed jojoba esters;
(c) at least one alkanolamine or a C₁-C₄ alkyl alkanolamine, selected from the group consisting of monoethanolamine (MEA), diethanolamine, triethanolamine, isopropanolamine, 2-amino-1-propanol (2A1P), 2-amino-1-butanol (2A1B), 2-amino-2-methyl-1-propanol (AMP), 2-dimethylamino-2-methyl-1-propanol (DMAMP), tris(hydroxymethyl)aminomethane (tromethamine), N,N-diethylethanolamine (DEEA), N,N-dimethylethanolamine (DMEA), N-methyldiethanolamine (MDEA), N-methylethanolamine (NMEA), and mixtures thereof;
(d) at least one oxidation dye, and
(e) at least one oxidizing agent.

In another embodiment, the composition for dyeing keratin fibers comprises, in a cosmetically acceptable medium:
(a) at least one hydrolyzed protein or peptide or amino acid, selected from the group consisting of hydrolyzed pea protein, a mixture comprising hydrolyzed soy protein, hydrolyzed wheat protein and hydrolyzed corn protein, and mixtures thereof;
(b) hydrolyzed jojoba esters;
(c) at least one alkanolamine or a C₁-C₄ alkyl alkanolamine, selected from the group consisting of monoethanolamine (MEA), diethanolamine, triethanolamine, isopropanolamine, 2-amino-1-propanol (2A1P), 2-amino-1-butanol (2A1B), 2-amino-2-methyl-1-propanol (AMP), 2-dimethylamino-2-methyl-1-propanol (DMAMP), tris(hydroxymethyl)aminomethane (tromethamine), N,N-diethylethanolamine (DEEA), N,N-dimethylethanolamine (DMEA), N-methyldiethanolamine (MDEA), N-methylethanolamine (NMEA), and mixtures thereof;
(d) at least one oxidation dye, and
(e) at least one oxidizing agent.

In another embodiment, the composition for dyeing keratin fibers comprises, in a cosmetically acceptable medium:
(a) at least one hydrolyzed protein or peptide or amino acid, selected from the group consisting of hydrolyzed pea protein, hydrolyzed soy protein, hydrolyzed wheat protein, hydrolyzed corn protein, glycine, taurine, and mixtures thereof;
(b) hydrolyzed jojoba esters;
(c) at least one alkanolamine or a C₁-C₄ alkyl alkanolamine, selected from the group consisting of monoethanolamine (MEA), 2-amino-1-propanol (2A1P), 2-amino-1-butanol (2A1B), 2-amino-2-methyl-1-propanol (AMP), and mixtures thereof;
(d) at least one oxidation dye, and
(e) at least one oxidizing agent.

In another embodiment, the composition for dyeing keratin fibers comprises, in a cosmetically acceptable medium:
(a) at least one hydrolyzed protein or peptide or amino acid, selected from the group consisting of hydrolyzed pea protein, a mixture comprising hydrolyzed soy protein, hydrolyzed wheat protein and hydrolyzed corn protein, and mixtures thereof;
(b) hydrolyzed jojoba esters;
(c) at least one alkanolamine or a C₁-C₄ alkyl alkanolamine, selected from the group consisting of monoethanolamine (MEA), 2-amino-1-propanol (2A1P), 2-amino-1-butanol (2A1B), 2-amino-2-methyl-1-propanol (AMP), and mixtures thereof;
(d) at least one oxidation dye, and
(e) at least one oxidizing agent.

In another embodiment, the composition for dyeing keratin fibers comprises, in a cosmetically acceptable medium:
(a) at least one hydrolyzed protein or peptide or amino acid, selected from the group consisting of hydrolyzed pea protein, a mixture comprising hydrolyzed soy protein, hydrolyzed wheat protein and hydrolyzed corn protein, and mixtures thereof;
(b) hydrolyzed jojoba esters;
(c) monoethanolamine (MEA);
(d) at least one oxidation dye, and
(e) at least one oxidizing agent.

In another embodiment, the composition for dyeing keratin fibers comprises, in a cosmetically acceptable medium:
(a) at least one hydrolyzed protein or peptide or amino acid, selected from the group consisting of a mixture comprising hydrolyzed soy protein, hydrolyzed wheat protein and hydrolyzed corn protein;
(b) hydrolyzed jojoba esters;
(c) monoethanolamine (MEA);
(d) at least one oxidation dye, and
(e) at least one oxidizing agent.

In another embodiment, the composition for dyeing keratin fibers comprises, in a cosmetically acceptable medium:
(a) at least one hydrolyzed protein or peptide or amino acid, selected from the group consisting of a mixture comprising hydrolyzed soy protein, hydrolyzed wheat protein and hydrolyzed corn protein;
(b) hydrolyzed jojoba esters;
(c) monoethanolamine (MEA);
(d) at least one oxidation dye, and
(e) hydrogen peroxide.

In one embodiment, the composition for dyeing keratin fibers comprises, in a cosmetically acceptable medium, based on the total weight of the composition:
(a) about 0.05-10 wt.% of at least one hydrolyzed protein or peptide or amino acid, and mixtures thereof,
(b) about 0.05-10 wt.% of hydrolyzed jojoba esters;
(c) about 0.05-10 wt.% of at least one alkanolamine, a C₁-C₄ alkyl alkanolamine, and mixtures thereof;
(d) about 0.001-10 wt.% of at least one oxidation dye, and
(e) about 0.05-10 wt.% of at least one oxidizing agent.

In an embodiment, the composition for dyeing keratin fibers comprises, in a cosmetically acceptable medium:
(a) about 0.05-10 wt.% of at least one hydrolyzed protein or peptide or amino acid, and mixtures thereof, selected from the group consisting of hydrolyzed silk protein, hydrolyzed pea protein, hydrolyzed soy protein, hydrolyzed wheat protein, hydrolyzed corn protein, and mixtures thereof;
(b) about 0.05-10 wt.% of hydrolyzed jojoba esters;
(c) about 0.05-10 wt.% of at least one alkanolamine, a C₁-C₄ alkyl alkanolamine, and mixtures thereof, selected from the group consisting of monoethanolamine (MEA), diethanolamine, triethanolamine, isopropanolamine, 2-amino-1-propanol (2A1P), 2-amino-1-butanol (2A1B), 2-amino-2-methyl-1-propanol (AMP), 2-dimethylamino-2-methyl-1-propanol (DMAMP), tris(hydroxymethyl)aminomethane (tromethamine), N,N-diethylethanolamine (DEEA), N,N-dimethylethanolamine (DMEA), N-methyldiethanolamine (MDEA), N-methylethanolamine (NMEA), and mixtures thereof;
(d) about 0.001-10 wt.% of at least one oxidation dye, and
(e) about 0.05-10 wt.% of at least one oxidizing agent.

In an embodiment, the composition for dyeing keratin fibers comprises, in a cosmetically acceptable medium:
(a) about 0.1-5 wt.% of at least one hydrolyzed protein or peptide or amino acid, selected from the group consisting of hydrolyzed pea protein, hydrolyzed soy protein, hydrolyzed wheat protein, hydrolyzed corn protein, glycine, alanine, serine, beta-alanine, taurine, sodium glycinate, sodium alaninate, sodium serinate, lithium beta-alanine, sodium taurate, and mixtures thereof;
(b) about 0.1-5 wt.% of hydrolyzed jojoba esters;
(c) about 0.1-5 wt.% of at least one alkanolamine or a C₁-C₄ alkyl alkanolamine, selected from the group consisting of monoethanolamine (MEA), diethanolamine, triethanolamine, isopropanolamine, 2-amino-1-propanol (2A1P), 2-amino-1-butanol (2A1B), 2-amino-2-methyl-1-propanol (AMP), 2-dimethylamino-2-methyl-1-propanol (DMAMP), tris(hydroxymethyl)aminomethane (tromethamine), N,N-diethylethanolamine (DEEA), N,N-dimethylethanolamine (DMEA), N-methyldiethanolamine (MDEA), N-methylethanolamine (NMEA), and mixtures thereof;
(d) about 0.001-10 wt.% of at least one oxidation dye, and
(e) about 0.5-8 wt.% of at least one oxidizing agent.

In another embodiment, the composition for dyeing keratin fibers comprises, in a cosmetically acceptable medium:
(a) about 0.15-3 wt.% of at least one hydrolyzed protein or peptide or amino acid, selected from the group consisting of hydrolyzed pea protein, hydrolyzed soy protein, hydrolyzed wheat protein, hydrolyzed corn protein, glycine, taurine, and mixtures thereof;
(b) about 0.15-3 wt.% of hydrolyzed jojoba esters;
(c) about 0.15-3 wt.% of at least one alkanolamine or a C₁-C₄ alkyl alkanolamine, selected from the group consisting of monoethanolamine (MEA), diethanolamine, triethanolamine, isopropanolamine, 2-amino-1-propanol (2A1P), 2-amino-1-butanol (2A1B), 2-amino-2-methyl-1-propanol (AMP), 2-dimethylamino-2-methyl-1-propanol (DMAMP), tris(hydroxymethyl)aminomethane (tromethamine), N,N-diethylethanolamine (DEEA), N,N-dimethylethanolamine (DMEA), N-methyldiethanolamine (MDEA), N-methylethanolamine (NMEA), and mixtures thereof;
(d) about 0.001-10 wt.% of at least one oxidation dye, and
(e) about 0.6-6 wt.% of at least one oxidizing agent.

In another embodiment, the composition for dyeing keratin fibers comprises, in a cosmetically acceptable medium:
(a) about 0.15-3 wt.% of at least one hydrolyzed protein or peptide or amino acid, selected from the group consisting of hydrolyzed pea protein, a mixture comprising hydrolyzed soy protein, hydrolyzed wheat protein and hydrolyzed corn protein, and mixtures thereof;
(b) about 0.15-3 wt.% of hydrolyzed jojoba esters;
(c) about 0.15-3 wt.% of at least one alkanolamine or a C₁-C₄ alkyl alkanolamine, selected from the group consisting of monoethanolamine (MEA), diethanolamine, triethanolamine, isopropanolamine, 2-amino-1-propanol (2A1P), 2-amino-1-butanol (2A1B), 2-amino-2-methyl-1-propanol (AMP), 2-dimethylamino-2-methyl-1-propanol (DMAMP), tris(hydroxymethyl)aminomethane (tromethamine), N,N-diethylethanolamine (DEEA), N,N-dimethylethanolamine (DMEA), N-methyldiethanolamine (MDEA), N-methylethanolamine (NMEA), and mixtures thereof;
(d) about 0.001-10 wt.% of at least one oxidation dye, and
(e) about 0.65-5 wt.% of at least one oxidizing agent.

In another embodiment, the composition for dyeing keratin fibers comprises, in a cosmetically acceptable medium:
(a) about 0.2-2 wt.% of at least one hydrolyzed protein or peptide or amino acid, selected from the group consisting of hydrolyzed pea protein, hydrolyzed soy protein, hydrolyzed wheat protein, hydrolyzed corn protein, glycine, taurine, and mixtures thereof;
(b) about 0.2-2 wt.% of hydrolyzed jojoba esters;
(c) about 0.2-2 wt.% of at least one alkanolamine or a C₁-C₄ alkyl alkanolamine, selected from the group consisting of monoethanolamine (MEA), 2-amino-1-propanol (2A1P), 2-amino-1-butanol (2A1B), 2-amino-2-methyl-1-propanol (AMP), and mixtures thereof;
(d) about 0.001-10 wt.% of at least one oxidation dye, and
(e) about 0.7-3 wt.% of at least one oxidizing agent.

In another embodiment, the composition for dyeing keratin fibers comprises, in a cosmetically acceptable medium:
(a) about 0.2-2 wt.% of at least one hydrolyzed protein or peptide or amino acid, selected from the group consisting of hydrolyzed pea protein, a mixture comprising hydrolyzed soy protein, hydrolyzed wheat protein and hydrolyzed corn protein, and mixtures thereof;
(b) about 0.2-2 wt.% of hydrolyzed jojoba esters;
(c) about 0.2-2 wt.% of at least one alkanolamine or a C₁-C₄ alkyl alkanolamine, selected from the group consisting of monoethanolamine (MEA), 2-amino-1-propanol (2A1P), 2-amino-1-butanol (2A1B), 2-amino-2-methyl-1-propanol (AMP), and mixtures thereof;
(d) about 0.001-10 wt.% of at least one oxidation dye, and
(e) about 0.7-3 wt.% of at least one oxidizing agent.

In another embodiment, the composition for dyeing keratin fibers comprises, in a cosmetically acceptable medium:
(a) about 0.2-2 wt.% of at least one hydrolyzed protein or peptide or amino acid, selected from the group consisting of hydrolyzed pea protein, a mixture comprising hydrolyzed soy protein, hydrolyzed wheat protein and hydrolyzed corn protein, and mixtures thereof;
(b) about 0.2-2 wt.% of hydrolyzed jojoba esters;
(c) about 0.2-2 wt.% of monoethanolamine (MEA);
(d) about 0.001-10 wt.% of at least one oxidation dye, and
(e) about 0.7-3 wt.% of at least one oxidizing agent.

In another embodiment, the composition for dyeing keratin fibers comprises, in a cosmetically acceptable medium:
(a) about 0.2-2 wt.% of at least one hydrolyzed protein or peptide or amino acid, selected from the group consisting of a mixture comprising hydrolyzed soy protein, hydrolyzed wheat protein and hydrolyzed corn protein;
(b) about 0.2-2 wt.% of hydrolyzed jojoba esters;
(c) about 0.2-2 wt.% of monoethanolamine (MEA);
(d) about 0.001-10 wt.% of at least one oxidation dye, and
(e) about 0.7-3 wt.% of at least one oxidizing agent.

In another embodiment, the composition for dyeing keratin fibers comprises, in a cosmetically acceptable medium:
(a) about 0.3-1.5 wt.% of at least one hydrolyzed protein or peptide or amino acid, selected from the group consisting of a mixture comprising hydrolyzed soy protein, hydrolyzed wheat protein and hydrolyzed corn protein;
(b) about 0.3-1.5 wt.% of hydrolyzed jojoba esters;
(c) about 0.3-1.5 wt.% of monoethanolamine (MEA);
(d) about 0.001-10 wt.% of at least one oxidation dye, and
(e) about 0.7 to lower than 1 wt.% of at least one oxidizing agent.

In another embodiment, the composition for dyeing keratin fibers comprises, in a cosmetically acceptable medium:
(a) about 0.3-1.5 wt.% of at least one hydrolyzed protein or peptide or amino acid, selected from the group consisting of a mixture comprising hydrolyzed soy protein, hydrolyzed wheat protein and hydrolyzed corn protein;
(b) about 0.3-1.5 wt.% of hydrolyzed jojoba esters;
(c) about 0.3-1.5 wt.% of monoethanolamine (MEA);
(d) about 0.001-7.5 wt.% of at least one oxidation dye, and
(e) about 0.7 to lower than 1 wt.% of hydrogen peroxide.

### Other Ingredients

The compositions for dyeing keratin fibers may contain a variety of other ingredients to enhance the beneficial properties of the dyeing compositions, as further described herein.

### 1. Thickening Agents

In an embodiment, the compositions for dyeing keratin fibers may contain one or more agents that will provide a thickening, or viscosity increasing, effect to the dyeing compositions. Suitable thickening agents include, but are not limited to, anionic, synthetic polymers, cationic synthetic polymers, naturally occurring thickeners, such as nonionic guar gums, scleroglucan gums or xanthan gums, gum arabic, gum ghatti, karaya gum, tragacanth gum, carrageenan gum, agar-agar, locust bean gum, pectins, alginates, starch fractions, and derivatives such as amylose, amylopectin, and dextrins, as well as cellulose derivatives such as, for example, methylcellulose, carboxyalkylcelluloses, and hydroxyalkylcelluloses, nonionic, fully synthetic polymers, such as polyvinyl alcohol or polyvinylpyrrolidinone; as well as inorganic thickeners, in particular phyllosilicates such as, for example, bentonite, in particular smectites, such as montmorillonite or hectorite. Suggested ranges are from about 0.001-20 wt.%, or about 0.005-15 wt.%, or about 0.01-12 wt.%, based on the total weight of the composition.

### 2. Preservatives

In an embodiment, the compositions for dyeing keratin fibers may contain one or more preservatives. Suitable preservatives include, but are not limited to, methyl, ethyl, and propyl paraben, hydantoins, and the like. Suggested ranges are about 0.0001-8 wt.%, or about 0.0005-7 wt.%, or about 0.001-5 wt.%, based on the total weight of the composition.

### 3. Chelating Agents

The dyeing compositions may also contain about 0.0001-5 wt.%, or 0.0005-3 wt.%, or 0.001-2 wt.%, based on the total weight of the composition, of one or more chelating agents which are capable of complexing with and inactivating metallic ions in order to prevent their adverse effects on the stability or effects of the composition. In particular, the chelating agent will chelate the metal ions found in the water and prevent these ions from interfering with the deposition and reaction of the dye with the hair fiber surface. Suitable chelating agents include, but are not limited to, sodium citrate; nitrogen-containing polycarboxylic acids, particularly Ethylenediaminetetraacetate (EDTA), and calcium, sodium, potassium or triethanolamine derivatives thereof, Hydroxyethyl Ethylenediamine Triacetic Acid (HEDTA), Ethylenediamine-N,N'-disuccinic acid (EDDS); and phosphonates, particularly 1-hydroxyethane-1,1-diphosphonate (HEDP), and/or ethylenediamine tetramethylene phosphonate (EDTMP), and/or diethylenetriamine pentamethylene phosphonate (DTPMP), or sodium salts thereof.

### 4. pH Adjusters

It may also be desirable to add small amounts of acids or buffering systems to adjust the pH of the dyeing compositions to the desired pH range of 7.1 to 11. Suitable acids include hydrochloric acid, phosphoric acid, citric acid, and the like. Suggested ranges of pH adjusters are from about 0.00001-8 wt.%, or about 0.00005-6 wt.%, or about 0 0001-5 wt.%, based on the total weight of the composition.

### 5. Reducing Agents

The dyeing compositions may also contain reducing agents to retard the reaction between oxidation bases and couplers and to prevent the initiation of the reaction in the packaging during the storage time. Sodium metabisulfite (sodium pyrosulfite) or sodium sulfite are typically used for this purpose.

### 6. Antioxidants

The dyeing compositions may also contain antioxidants to avoid the reaction beginning before the addition of the oxidizing agent ( e.g., hydrogen peroxide).

Water-soluble antioxidants such as erythorbic acid or oil-soluble antioxidants such as T-butylquinone are typically used.

### 7. Opacifiers

The dyeing compositions may also contain opacifiers. Suitable opacifiers include, but are not limited to, titanium dioxide (TiO2), latex, styrene/PVP, styrene/acrylamide copolymers, and mixtures thereof.

### 8. Pigments

The dyeing compositions may also contain inorganic pigments. Suitable inorganic pigments include, but are not limited to, those having a color index number as listed in the CTFA dictionary, 10th edition, 2004, hereby incorporated by reference.

### 9. Direct dyes

The dyeing compositions may also contain direct dyes. Direct dye, also called substantive dye, is a dye that adheres to its substrate, by non-ionic forces, e.g., without help from other chemicals. For purposes of this disclosure dyes include direct dyes selected from anionic, cationic and non-ionic nitro dyes.

Examples of anionic direct dyes include, but are not limited to, Bromophenol Blue, Tetrabromophenol Blue, Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 5, FD&C Yellow No. 6, FD&C Blue 1, Food Black 1, Food Black 2, Disperse Black 9 and Disperse Violet 1 and their alkali metal salts such as sodium, potassium.

Examples of cationic dyes include, but are not limited to, Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12, Basic Red 22, Basic Red 76, Basic Violet 1, Basic Violet 2, Basic Violet 3, Basic Violet 10, Basic Violet 14, Basic Yellow 57, Basic red 51, Basic Yellow 87 and Basic Orange 31.

Examples of non-ionic nitro dyes (HC dyes) include, but are not limited to, HC Blue No. 2, HC Blue No. 4, HC Blue No. 5, HC Blue No. 6, HC Blue No. 7, HC Blue No. 8, HC Blue No. 9, HC Blue No. 10, HC Blue No. 11, HC Blue No. 12, HC Blue No. 13, HC Blue No. 15, Blue No. 16, HC Blue No. 18, HC Brown No. 1, HC Brown No. 2, HC Green No. 1, HC Orange No. 1, HC Orange No. 2, HC Orange No. 3, HC Orange No. 5, HC Red BN, HC Red No. 1, HC Red No. 3, HC Red No. 7, HC Red No. 8, HC Red No. 9, HC Red No. 10, HC Red No. 11, HC Red No. 13, HC Red No. 14, HC Red No. 18, HC Red No. 54, HC Violet BS, HC Violet No. 1, HC Violet No. 2, HC Yellow No. 2, HC Yellow No. 4, HC Yellow No. 5, HC Yellow No. 6, HC Yellow No. 7, HC Yellow No. 8, HC Yellow No. 9, HC Yellow No. 10, HC Yellow No. 11, HC Yellow No. 12, HC Yellow No. 13, HC Yellow No. 14, HC Yellow No. 15, 2-Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

### 10. Fragrance or perfumes

For purposes of this disclosure, the term "fragrance" as used herein refers to any substance, natural or synthetic, used to impart an odour to a product.

For purposes of this disclosure, the term "perfume" as used herein refers to any mixture of fragrant essential oils and aroma compounds, fixatives, and solvents used to give the human body, objects, and living spaces a lasting and pleasant smell.

### 11. Nonionic surfactants

Non-limiting examples of nonionic surfactants in the context of the present disclosure include, but are not limited to, alkoxylated C₆-C₂₄ fatty alcohols, polyethoxylated or polypropoxylated fatty acids, alkylphenols, alpha-diols or alcohols having a fatty chain containing, for example, 8 to 18 carbon atoms, ethylene oxide or propylene oxide groups to range in particular from 2 to 50; copolymers of ethylene oxide and of propylene oxide, polyethoxylated fatty amides having from 2 to 30 mol of ethylene oxide; polyglycerolated fatty amides containing on average 1 to 5, or 1.5 to 4, glycerol groups; polyethoxylated fatty amines having 2 to 30 mol of ethylene oxide; oxyethylenated fatty acid esters of sorbitan having from 2 to 30 mol of ethylene oxide; fatty acid esters of sucrose; fatty acid esters of polyethylene glycol; alkylpolyglycosides; N-alkylglucamine derivatives; amine oxides such as (C₁₀-C₁₄) alkylamine oxides or N-acylaminopropylmorpholine oxides.

The term "alkoxylated C₆-C₂₄ fatty alcohol" as used herein refers to ethoxylated or propoxylated C₆-C₂₄ fatty alcohols.

Ethoxylated alcohols (also called polyethyleneglycol ethers or PEG ethers) are produced from the reaction of fatty alcohols with ethylene oxide (EO).

Alkoxylated alcohols (also called polypropyleneglycol ethers or PPG ethers) are produced from the reaction of fatty alcohols with propylene oxide (PO).

Examples of suitable alkoxylated C₆-C₂₄ fatty alcohols include, but are not limited to, ethoxylated palmytil (cetyl) alcohol, ethoxylated palmitoyl alcohol, ethoxylated stearyl alcohol, ethoxylated cetearyl alcohol, ethoxylated isostearyl alcohol, ethoxylated 2-octyldodecanol, ethoxylated 2-ethylhexanoyl alcohol, ethoxylated oleyl alcohol, and mixtures thereof.

Non-limiting examples of alkoxylated C₆-C₂₄ fatty alcohols that may be used according to the present disclosure, include the adducts of ethylene oxide, in particular those containing from 2 to 50 ethylene oxide units, with cetyl alcohol, stearyl alcohol, cetearyl alcohol, isostearyl alcohol, octyldodecanol, 2-ethylhexanoyl alcohol, oleyl alcohol, and mixtures thereof, such as Ceteth-4, Ceteth-5, Ceteth -6, Ceteth-10, Ceteth-12, Ceteth-14, Ceteth-15, Ceteth-16, Ceteth-20, Ceteth-24, Ceteth-25, Ceteth-30, Ceteth-45, Ceteareth-4, Ceteareth-5, Ceteareth-6, Ceteareth-7, Ceteareth-8, Ceteareth-9, Ceteareth-10, Ceteareth-11, Ceteareth-12, Ceteareth-13, Ceteareth-14, Ceteareth-15, Ceteareth-16, Ceteareth-17, Ceteareth-18, Ceteareth-20, Ceteareth-22, Ceteareth-23, Ceteareth-24, Ceteareth-25, Ceteareth-27, Ceteareth-28, Ceteareth-29, Ceteareth-30, Ceteareth-33, Ceteareth-34, Ceteareth-40, Ceteareth-50, Steareth-5, Steareth-8, Steareth-14, Steareth-16, Steareth-21, Steareth-25, Steareth-27, Steareth-30, Steareth-40, Steareth-50, Isosteareth-2, Isoteareth-20, Oleth-2, Oleth-3, Oleth-4, Oleth-5, Oleth-6, Oleth-7, Oleth-8, Oleth-9, Oleth-10, Oleth-11, Oleth-12, Oleth-15, Oleth-16, Oleth-20, Oleth-23, Oleth-25, Oleth-30, Oleth-40, Oleth-44, Oleth-50, and mixtures thereof.

### 12. Anionic surfactants

Non-limiting examples of anionic surfactants include, but are not limited to, alkyl ether carboxylates, alkyl sulfates, alkyl ether sulfates, amide ether sulfates, alkyl glyceride sulfates, olefin sulfonates, alkyl-aryl sulfonates, sulfosuccinates, sulfo fatty acid esters, fatty acid isethionates, fatty acid taurides, phosphate esters, acyl glutamates, acyl peptides, acyl sarcosides, and mixtures thereof. Typically, the at least one anionic surfactant is selected from alkyl ether carboxylates, alkyl ether sulfates, olefin sulfonates, and mixtures thereof.

Alkyl ether carboxylates are obtained by ethoxylation and subsequent carboxymethylation of fatty alcohols (preferably having 6 to 24 carbon atoms). The process is divided into two steps. The first one is the ethoxylation of alcohols under standard conditions known by the skilled in the art. However, one may also start from commercially available ethoxylated alcohols.

In the second step, the ethoxylated alcohols are reacted with a strong base, such as sodium or potassium hydroxide, in presence of a reducing agent, e.g., sodium borohydride, to obtain the corresponding alkoxylate, which is carboxymethylated with sodium monochloroacetate (SMCA).

Optionally, the crude alkyl ether carboxylate can be purified by first converting the crude products with hydrochloric acid or sulphuric acid into the free acid. Said acid is washed and after that it is converted into the corresponding salt.

Examples of suitable alkyl ether carboxylates include, but are not limited to, Potassium Laureth-4 Carboxylate, Sodium Laureth-4 Carboxylate, Sodium Laureth-5 Carboxylate, Sodium Laureth-6 Carboxylate, and Sodium Laureth-11 Carboxylate.

Alkyl ether sulfates are obtained by the sulfonation of an ethoxylated alcohol. Examples of suitable alkyl ether sulfates include, but are not limited to, Ammonium Laureth Sulfate, Monoethanolamine (MEA)-Laureth Sulfate, Monoisopropanolamine (MIPA)-Laureth Sulfate, Sodium Laureth Sulfate, and Triethanolamine (TEA)-Laureth Sulfate; particularly Sodium Laureth Sulfate having an average degree of ethoxylation of about 1 to 3.

Olefin sulfonates can be produced by sulfonation of an alpha olefin by means of uncomplexed sulfur trioxide, followed by neutralization of the acid reaction mixture in conditions such that any sultones which have been formed in the reaction are hydrolyzed to give the corresponding hydroxy-alkanesulfonates. Examples of suitable olefin sulfonates include, but are not limited to, C₁₄-C₁₆ Olefin Sulfonate salts, such as Sodium C₁₄-C₁₆Olefin Sulfonate.

Typically, in the composition for dyeing keratin fibers, the total amount of (a) the at least one anionic surfactant is in the range of about 0.1 to about 25 wt.%, or about 0.3 to about 15 wt. %, or about 0.5 to about 10 wt.%, based on the total weight of the composition.

### 13. Cationic surfactants

Non-limiting examples of cationic surfactants include, but are not limited to, alkylimidazolines, tetra alkyl(-aryl) quaternary ammonium salts (quats), heterocyclic ammonium salts, quaternized alkyl polyglycosides, quaternized derivatives of polyalkanolamine esters (esterquats), and mixtures thereof. Typically, the at least one cationic surfactant is selected from C₆-C₂₄ alkyl trimethyl quaternary ammonium salts, C₆-C₂₄ alkyl dimethyl benzyl quaternary ammonium salts, C₆-C₂₄ dialkyl dimethyl quaternary ammonium salts, and mixtures thereof.

Examples of suitable C₆-C₂₄ alkyl trimethyl quaternary ammonium salts include, but are not limited to, Laurtrimonium bromide, Laurtrimonium chloride, Myrtrimonium bromide, Myrtrimonium chloride, Cetrimonium bromide, Cetrimonium chloride, Cetrimonium metosulfate, Steartrimonium bromide, Steartrimonium chloride, Steartrimonium methosulfate, Behentrimonium bromide, Behentrimonium chloride, Behentrimonium methosulfate, Ceteartrimonium chloride, Cocotrimonium chloride, Cocotrimonium methosulfate, Soytrimonium chloride, Octyl dodecyl trimethyl ammonium chloride, Dodecyl hexadecyl trimethyl ammonium bromide, Dodecyl hexadecyl trimethyl ammonium chloride, and mixtures thereof.

Examples of suitable C₆-C₂₄ dialkyl dimethyl quaternary ammonium salts include, but are not limited to, Benzalkonium chloride, benzyl-C₁₀-₁₆-alkyldimethylammonium chloride, benzyl-C₁₂-₁₄-alkyldimethylammonium chloride and mixtures thereof.

Examples of suitable C₆-C₂₄ dialkyl dimethyl quaternary ammonium salts include, but are not limited to, Didecyldimonium chloride, Dilauryldimonium chloride, Distearyldimonium chloride, and mixtures thereof.

Examples of suitable esterquats include, but are not limited to, Behenoyl PG-Trimonium Chloride, Dioleoylethyl Hydroxyethylmonium Methosulfate, and mixtures thereof.

### 14. Amphoteric or zwitterionic surfactants

Non-limiting examples of amphoteric or zwitterionic surfactants include, but are not limited to, aliphatic secondary or tertiary amine derivatives in which the aliphatic radical is a linear or branched chain containing 8 to 18 carbon atoms and containing at least one water-soluble anionic group (for example carboxylate, sulphonate, sulphate, phosphate or phosphonate); (C₈-C₂₀) alkylbetaines, sulphobetaines, (C₈-C₂₀) alkylamido (C₁-C₆) alkylbetaines or (C₈-C₂₀) alkylamido (C₁-C₆) alkylsulphobetaines; amine derivatives, disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid, and cocoamphodipropionic acid.

### 15. C₆-C₂₄ fatty alcohols

Non-limiting examples of C₆-C₂₄ fatty alcohols include, but are not limited to C₆-C₂₄ fatty alcohols from vegetable fats and oils and include cotton, safflower, coconut, rapeseed, linseed, palm, palm kernel, sunflower, olein, olive, olive pomace, castor oil, soy, tall oil, etc, possibly totally or partially hydrogenated, as well as purified or synthetic fatty alcohols such as caproyl alcohol, capryl alcohol, capric alcohol, lauryl alcohol, myristyl alcohol, palmytil (cetyl) alcohol, palmitoyl alcohol, stearyl alcohol, isostearyl alcohol, 2-octyldodecanol, 2-ethylhexanoyl alcohol, oleyl alcohol, ricinoleyl alcohol, elaidyl alcohol, petroselinic alcohol, linoleyl alcohol, linolenyl alcohol, arachidyl alcohol, gadoleyl alcohol, behenyl alcohol and erucyl alcohol, or technical grade mixtures.

Examples of suitable C₆-C₂₄ fatty alcohols include, but are not limited to, lauryl alcohol, myristyl alcohol, palmytil (cetyl) alcohol, palmitoyl alcohol, stearyl alcohol, cetearyl alcohol, isostearyl alcohol, 2-octyldodecanol, 2-ethylhexanoyl alcohol, oleyl alcohol, behenyl alcohol, erucyl alcohol, and mixtures thereof, in particular palmytil (cetyl) alcohol, palmitoyl alcohol, stearyl alcohol, cetearyl alcohol, isostearyl alcohol, 2-octyldodecanol, 2-ethylhexanoyl alcohol, oleyl alcohol, behenyl alcohol, erucyl alcohol, and mixtures thereof.

### 16. Petroleum hydrocarbons (mineral oils, paraffins and waxes)

For purposes of this disclosure petroleum hydrocarbons are represented as mineral oils, paraffins and waxes based on petroleum. Examples include, but are not limited to, hard paraffin, liquid paraffin (Liquid Petrolatum or Paraffinum Liquidum), light liquid paraffin (Light Liquid Petrolatum or Paraffinum Perliquidium), white soft paraffin (White Petrolatum), yellow soft paraffin (Yellow Petrolatum), macrocrystalline paraffin waxes (which are mixtures which consist mainly of saturated C₁₈-C₃₀ hydrocarbons and smaller amounts of iso-alkanes and cycloalkanes with a molecular weight comprised between 250 and 450 g/mol and, although they are solids at room temperature, they have low melting points, usually comprised between 40°C and 60°C), microcrystalline paraffin waxes (which consist of C₄₀-C₅₅ compounds which contain, in addition to normal hydrocarbons, included in the group are iso-alkanes and naphtenes with long alkyl side-chains, the iso-alkanes forming microcrystals, the microcrystalline paraffin waxes having mean molecular weights comprised between 500 and 800 g/mol, being solids at room temperature, and having melting points comprised between 60°C and 90°C), or mixtures thereof.

### 17. Vegetable fats and oils

For purposes of this disclosure vegetable fats and oils are linear and/or branched esters, linear or branched, saturated and/or unsaturated alkanecarboxylic acids with a chain length of 1 up to 30 carbon atoms, saturated and/or unsaturated alcohols with a chain length of 1 up to 30 carbon atoms; or linear and/or branched esters of aromatic carboxylic acids, or saturated and/or unsaturated alcohols with a chain length of 1 up to 30 carbon atoms. These oils can be selected from the group consisting of for example isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 2-ethylhexyl laurate, 2-ethylhexyl palmitate, 2-ethylhexyl cocoate, 2-hexyldecyl stearate, 2- ethylhexyl isostearate, 2-octyldodecyl palmitate, cetyl palmitate, stearyl palmitate, oleyl palmitate, oleyl oleate, oleyl erucate, erucyl oleate, erucyl erucate, as well as synthetic, semisynthetic and natural mixtures esters, such as jojoba oil (a natural mixture of esters of monounsaturated monocarboxylic acids with a C₁₈-C₂₄ chain with also monounsaturated monoalcohols and with a long C₁₈-C₂₄ chain).

Other examples of vegetable fats and oils include ester oils such as sugar esters or diesters of C₁₂-C₂₄ fatty acids. The term "sugar" means compounds comprising several alcohol functions, with or without an aldehyde or ketone function, and which comprise at least 4 carbon atoms. These sugars may be monosaccharides, oligosaccharides or polysaccharides.

Non-limiting examples of sugars that may be used according to the present disclosure, include sucrose (or saccharose), glucose, galactose, ribose, fucose, maltose, fructose, mannose, arabinose, xylose and lactose, and derivatives thereof, including for example, alkyl derivatives such as methyl derivatives, for instance methylglucose. Non-limiting examples of the sugar esters of fatty acids that may be used according to this disclosure include those from the group comprising esters or mixtures of esters of sugars described above and of linear or branched, saturated or unsaturated C₁₂-C₂₄ fatty acids.

The esters may be chosen from mono-, di-, tri-, tetraesters and polyesters, and mixtures thereof. These esters may be chosen from, for example, but not limited to, oleates, laurates, palmitates, myristates, behenates, cocoates, stearates, linoleates, linolenates, caprates and arachidonates, or mixtures thereof such as oleo-palmitate, oleo-stearate and palmito-stearate mixed esters. It will be noted that the sucrose, glucose or methylglucose monoesters and diesters and for example sucrose, glucose or methylglucose mono- or dioleates, stearates, behenates, oleopalmitates, linoleates, linolenates and oleostearates, constitute sugar esters or diesters of C₁₂-C₂₄ fatty acids that are suitable in the context of the present disclosure. A particular non-limiting example that may be mentioned is methylglucose dioleate.

Other suitable oils of the type of esters of saturated alkane carboxylic acids and alcohols are fatty acid methyl esters, such as C₆-C₂₄ fatty acid methyl esters from animal and vegetable fats and oils such as cotton, safflower, coconut, rapeseed, linseed, palm, palm kernel, sunflower, olein, olive, olive pomace, castor oil, soy, tall oil, etc, possibly totally or partially hydrogenated, as well as purified or synthetic fatty acids such as caproic acid, caprylic acid, capric acid, lauric acid, myristic acid, palmitic acid (cetylic acid), palmitoleic acid, stearic acid, isostearic acid, 2-ethylhexanoic acid, oleic acid, ricinoleic acid, elaidic acid, petroselinic acid, linoleic acid, linolenic acid, arachidic acid, gadoleic acid, behenic acid and erucic acid, or mixtures thereof.

Other suitable vegetable fats and oils according to the present disclosure are fatty acid triglycerides, including for example, linear and/or branched triglycerin esters, saturated and/or unsaturated alkanecarboxylic acids with a chain length of 6 up to 24 carbon atoms, preferably of 10 up to 18 carbon atoms. The fatty acids esterifying the different positions of glycerin can be different, giving rise to a large amount of possible combinations, including positional combinations. The position of the different fatty acids in natural triglycerides is not random, but rather it depends on the origin of the fat. The most simple triglycerides are those constituted by a sole fatty acid.

Fatty acid triglycerides can be chosen, for example, from the group consisting of synthetic, semi-synthetic and natural oils, as for example avocado oil, almond oil, hazelnut oil, babassu palm oil, borage oil, peanut oil, canola oil, hemp oil, milk thistle oil, safflower oil, chufa oil, coconut oil, rapeseed oil, black cumin oil, wheat germ oil, sunflower oil, linseed oil, macadamia nut oil, corn oil, walnut oil, olive oil and its byproducts such as olive pomace oil, palm oil and its fractions such as palm olein and palm stearin, evening primrose oil, rosehip oil, castor oil, rice bran oil, apricot kernel oil, cottonseed oil, pumpkinseed oil, palm kernel oil and its fractions such as palm kernel olein and palm kernel stearin, grape seed oil, sesame oil, soy oil, cocoa butter, shea butter and the like.

### 18. Natural waxes

Natural waxes according to the present disclosure, include but are not limited to, candelilla wax, carnauba wax, Japan wax, esparto wax, cork wax, guaruma wax, rice wax, sugar cane wax, ouricury wax, montan wax, beeswax, shellac wax, espermaceti, wool lanolin (wax), uropygial fat wax, ceresin waxes, peat waxes, ozokerite, as well as chemically modified waxes (hard waxes) for example, montan wax esters, waxes obtained by the Fischer-Tropsch process, hydrogenated jojoba waxes and synthetic waxes.

### 19. Silicones

For purposes of the present disclosure silicones include, cyclic and/or linear silicones, which can be found as monomers generally characterized by structural elements such as: 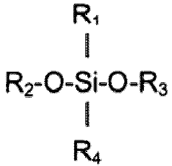 where the silicon atoms can be substituted by alkyl or aryl radicals equal or different, represented here generally by R₁-R₄ groups.

Linear silicones with siloxane units suitable according to the present disclosure are generally characterized by structural elements such as: 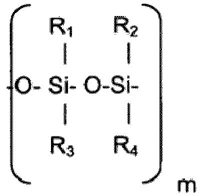 where the silicon atoms can be substituted by alkyl or aryl radicals equal or different, represented here in general by R₁-R₄ groups (meaning the number of different radicals is not necessarily limited to 4), m can take values from 2 to 200.000.

Cyclic silicones suitable according to the present disclosure are generally characterized by structural elements such as: 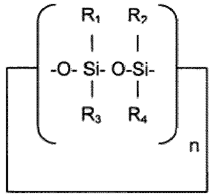 where the silicon atoms can be substituted by alkyl or aryl radicals equal or different, represented here generally by R₁-R₄ groups (meaning the number of different radicals is not necessarily limited to 4), n can take values of 3/2 to 20. Fractional values of n indicate that it may be odd numbers of siloxane groups present in the ring.

Specific examples include a cyclic methyl siloxane having the formula [(CH₃)₂SiO]x in which x is 3-6, or short chain linear methyl siloxanes having the formula ((CH₃)₂SiO[(CH₃)₂SiO]_{y}Si(CH₃)₃ in which y is 0-5.

Some suitable cyclic methyl siloxanes are hexamethylcyclotrisiloxanes (D3), octamethylcyclotetrasiloxane (D4), decamethylcyclopentasiloxane (D5) (cyclomethicone) and dodecamethylcyclohexasiloxane (D6).

Some suitable short linear methyl siloxane are hexamethyldisiloxane (MM), octamethyltrisiloxane (MDM), decamethyltetrasiloxane (MD2M), dodecamethylpentasiloxane (MD3M), tetradecamethylhexasiloxane (MD4M), and hexadecamethylheptasiloxane (MD5M).

Furthermore, long chain linear siloxanes such as phenyltrimethicone, bis(phenylpropyl)dimethicone, dimethicone, dimethiconol, cetyldimethicone and behenoxy dimethicone are also suitable silicones according to the present disclosure.

### 20. Cationic Polymers

The term "cationic polymer" as used herein refers to a macromolecule (polymer) that contains at least one monomer bearing a quaternary ammonium group.

Examples of suitable cationic polymers include, but are not limited to, those polymers known by their International Nomenclature for Cosmetic Ingredients (INCI) name as Polyquaternium. Typical examples of those are Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-42, Polyquaternium-43, Polyquaternium-44, Polyquaternium-45, Polyquaternium-46, Polyquaternium-47, Polyquaternium-48, Polyquaternium-49, Polyquaternium-50, Polyquaternium-51, Polyquaternium-52, Polyquaternium-53, Polyquaternium-54, Polyquaternium-55, Polyquaternium-56, Polyquaternium-57, Polyquaternium-58, Polyquaternium-59, Polyquaternium-60, Polyquaternium-61, Polyquaternium-62, Polyquaternium-63, Polyquaternium-64, Polyquaternium-65, Polyquaternium-66, Polyquaternium-67, Polyquaternium-68, Polyquaternium-69, Polyquaternium-70, Polyquaternium-71, Polyquaternium-72, Polyquaternium-73, Polyquaternium-74, Polyquaternium-75, Polyquaternium-76, Polyquaternium-77, Polyquaternium-78, Polyquaternium-79, Polyquaternium-80, Polyquaternium-81, Polyquaternium-82, Polyquaternium-83, Polyquaternium-84, Polyquaternium-85, Polyquaternium-86, Polyquaternium-87, Polyquaternium-88, Polyquaternium-91, Polyquaternium-92, Polyquaternium-94, Polyquaternium-95, Polyquaternium-96, Polyquaternium-98, Polyquaternium-99, Polyquaternium-100, Polyquaternium-102, Polyquaternium-104, Polyquaternium-109, Polyquaternium-110, Polyquaternium-111, Polyquaternium-112, Polyquaternium-113 and Polyquaternium-114. Other suitable cationic polymers include those known by their INCI name as Guar hydroxypropyl trimonium chloride, Polyacrylamidopropyltrimonium Chloride, Polymethacrylamidopropyltrimonium Chloride and Polymethacrylamidopropyltrimonium Methosulfate.

### 21. Non-ionic polymers

For purposes of this disclosure, non-limiting examples of non-ionic polymers include, but are not limited to:
- nonionic guar gums and modified nonionic guar gums may be for example, but are not limited to, those modified with C₁-C₆ hydroxyalkyl groups;
- biopolysaccharide gums of microbial origin such as scleroglucan gum or xanthan gum;
- gums derived from plant exudates, such as gum arabic, ghatti gum, karaya gum, tragacanth gum, carrageenan, agar and carob gum;
- pectins;
- alginates;
- starches;
- hydroxy (C₁-C₆) alkylcelluloses and carboxy (C₁-C₆) alkylcelluloses;
- celluloses modified with groups comprising at least one fatty chain; non-limiting mention may be made, for example, of hydroxyethylcelluloses modified with groups comprising at least one fatty chain, such as alkyl, arylalkyl or alkylaryl groups, or mixtures thereof, and wherein the alkyl groups are, for example, C₈-C₂₂; or those modified with polyalkylene glycol alkylphenyl ether groups;
- hydroxypropyl guars modified with groups comprising at least one C₈-C₂₂ fatty chain,
- copolymers of vinylpyrrolidone and of hydrophobic monomers comprising a fatty chain, such as vinylpyrrolidone/hexadecene copolymer or vinylpyrrolidone/eicosene copolymer;
- copolymers of C1-C6 alkyl acrylates or methacrylates and of amphiphilic monomers comprising at least one fatty chain;
- copolymers of hydrophilic acrylates or methacrylates and of hydrophobic monomers comprising at least one fatty chain, such as the polyethylene glycol methacrylate/lauryl methacrylate copolymer;
- polymers with an aminoplast ether skeleton comprising at least one fatty chain;
- polyurethane polyethers comprising in their chain both hydrophilic blocks, for example of polyoxyethylenated nature, and hydrophobic blocks that may be aliphatic blocks alone and/or cycloaliphatic and/or aromatic blocks.

### 22. Amphoteric polymers

For purposes of this disclosure, non-limiting examples of amphoteric polymers include, but are not limited to, amphoteric polysaccharides such as Carboxymethylchitosan or N-[(2'-Hydroxy-2',3'-dicarboxy)ethyl]chitosan; Amphoteric Urethanes; Modified Potato Starch; Methacryloyl Ethyl Betaine/Acrylates Copolymer; Acrylates/Lauryl Acrylate/Stearyl Acrylate/Ethylamine Oxide Methacrylate; and Acrylic acid/(meth)acrylamidopropyl-trimethylarnmonium chloride/stearyl methacrylate terpolymer.

### 23. Anionic polymers

The compositions for treating keratin fibers may include one or more anionic polymers. Non-limiting examples of anionic polymers include, but are not limited to, those polymers known by their International Nomenclature for Cosmetic Ingredients (INCI) name as Shellac, Acrylates Copolymer, Acrylates/C1-2 Succinates/Hydroxyacrylates Copolymer, Acrylic Esters (and) Methacrylic Esters Copolymer, AMP-Acrylates/Allyl Methacrylate Copolymer, Diglycol/Cyclohexanedimethanol/Isophtalates/Sulfoisophtalates Copolymer, Diglycol/Isophtalates/Sulfoisophtalates Copolymer, Isobutylene Ethylmaleimide/Hydroxyethylmaleimide Copolymer, Glycerin and Diglycol/Cyclohexanedimethanol/Isophtalates/Sulfoisophtalates Copolymer, Methacrylate Acid/Sodium Acrylamidomethyl Propane Sulfonate Copolymer, Octylacrylamide/Acrylates/Butylaminoethylmethacrylate Copolymer, Polyurethane (and) Acrylates Copolymer, PVM/MA Copolymer, PVP/Ethyl Methacrylate/Methacrylic Acid Terpolymer, PVP/Polycarbamyl Polyglycol Ester, VA/Crotonates Copolymer, VA/Crotonates/Vinyl Neodecanoate Copolymer, VP/Acrylates/Lauryl Methacrylate Copolymer, and their cosmetically acceptable salts.

### 24. Other additives

The compositions for treating keratin fibers may include one or more additives other than those described above. Non-limiting examples of additives different from those listed above include, but are not limited to, Vitamins and Provitamins, such as beta-carotene, retinal, retinol, retinoic acid, biotin, panthenol, panthenyl ethyl ether, ascorbic acid, ascorbyl glucoside, ascorbyl palmitate, ascorbyl phosphate, tocopherol, tocopheryl acetate, tocopheryl glucoside, tocopheryl palmitate, and the like; hair strengthening agents such as maleic acid, hydroxypropylgluconamide (and) hydroxypropylammonium gluconate, and the like; antioxidants (ingredients employed in cosmetics to prevent or retard product spoilage from rancidity or deterioration from reaction with oxygen); moisturizers such as allantoin; natural extracts (substances or active ingredients with desirable properties that are removed from a plant, flower, alga, fungus or bacteria); UV filters (ingredients used to absorb or reflect the UV rays that are contained in sun light or in artificial light); or mixtures thereof.

No particular limitation is imposed on the form of the compositions for dyeing keratinous fibers of the present disclosure. Examples include transparent liquid, emulsion, cream, mousse, gel, paste, aerosol, and aerosol foam.

Typically, the compositions for dyeing keratin fibers of the present disclosure present a viscosity in the range of about 1,500-70,000 mPa.s, or in the range of about 1,500-50,000 mPa.s, or in the range of about 2,000-30,000 mPa.s, measured at 25°C, using a Brookfield Dial Reading Viscometer LVT with spindle F at 12 rpm.

The pH of the compositions for dyeing keratin fibers of the present disclosure may be in the range of about 5.5 to about 8.0, or in the range of about 6.0 to about 8.0, or in the range of about 6.0 to about 7.5, measured at 25°C.

### The process for dyeing keratin fibers

Another embodiment of the present disclosure refers to a process for dyeing keratin fibers comprising the steps of:
i) applying a dyeing composition as defined above to the keratin fibers for a period which is sufficient to obtain the dyeing effect; and
ii) removing the dyeing composition from the keratin fibers by rinsing with water.

The term "period which is sufficient to obtain the dyeing effect" refers to the time needed for the hair to develop the desired color. Typically, the dyeing composition is allowed to remain in the hair for a period of 5-50 minutes, or for a period of 10-40 minutes, at a temperature ranging from 15-50°C.

The composition for dyeing keratin fibers is typically obtained by mixing a tint composition (composition comprising the oxidation dyes) and an oxidizing composition (typically a hydrogen peroxide composition) to form a ready-to-use composition, i.e. the dyeing composition which is applied to the keratin fibers.

The mixing ratio of the tint composition and the oxidizing composition (i.e. hydrogen peroxide composition) may be about 1:1 to 1:3, or about 1:1 to 1:2.5.

It should be noted that the mixing ratio of the composition for dyeing keratin fibers of the disclosure and the hydrogen peroxide composition is very much dependent on the level of dyeing effect targeted. The above-mentioned ratios are general and in case somewhat different mixing ratios are needed simply because of reaching higher dyeing level than usual levels, such mixing ratio should still be understood being within the scope of the present disclosure.

Typically, the oxidizing composition is in the form of an aqueous solution (aqueous hydrogen peroxide composition). The term "aqueous" means that the hydrogen peroxide composition comprises more than about 5 wt.% of water, or more than about 10 wt.% of water, or more than 20 wt.% of water.

The concentration of hydrogen peroxide may range from about 0.5 to 50 wt.%, or from about 1.0 to 40 wt.%, or from about 1.0 to 15 wt.%, or from about 1.0 to about 6 wt.%. As a function of the desired degree of lightening, the oxidizing composition may also comprise an oxidizing agent preferably chosen from peroxygenated salts.

Another embodiment of the present disclosure refers to a process for dyeing keratin fibers as described above, wherein the process is intended for reducing the damage of the keratin fibers.

Application of the composition for dyeing keratin fibers to the hair may be undertaken in several ways. Application of the composition for dyeing keratin fibers may take place on the whole head of hair of an end user. As used herein, the "whole head of hair" means that the hair all over the head from the root of the hair to the tip of the hair is included in the application process.

By contrast, the application of the composition for dyeing keratin fibers may take place only on the root portion of the hair. The application to the root portion of the hair may still be over the entire head of the end user, but application of the composition for dyeing keratin fibers is applied only to the section of hair closest to the head (root portion), which is between about 0.01 mm to about 40 mm from the scalp of the head. After application to the root portion, the composition for dyeing keratin fibers may be applied to the rest of the hair at a later stage to prevent over processing of the hair in the lengths and ends.

Also, application may take place on a portion of hair. Application of a portion of hair is commonly referred to as highlighting or lowlighting. The portion of hair may be physically separated from the whole head of hair in a hair bundle or may be a smaller portion of hair than the whole head of hair. A hair bundle may be physically separated from a whole head of hair by a device including a plastic cap through which hair bundles are formed when hair is pulled through orifices in the plastic cap, metal foils encompassing a hair bundle, strand separators applied to hair at the root portion, or similar devices.

The process for dyeing keratin fibers also may further comprise working the composition for dyeing keratin fibers into the treated hair surface by hand or by a tool for a period which is sufficient to obtain the dyeing effect to ensure uniform application to the entire treated hair surface.

The composition for dyeing keratin fibers can be applied on hair via an applicator bottle or brush. It can be used on full head or partly on single strands (highlight application) as common highlight applicator foils, caps and special applicators can be used, but also freehand techniques such as balayage, with brush and/or combs can be possible. The composition can also be applied as a mousse via a manual spray, a pressurized container or an aerosol mousse.

### The multi-compartment device

Another embodiment of the present disclosure refers to a multi-compartment device for dyeing keratin fibers comprising at least two compartments packaged separate from one another, wherein
(i) one compartment comprises a tint composition comprising:
   (a) at least one hydrolyzed protein or peptide or amino acid, and mixtures thereof,
   (b) hydrolyzed jojoba esters;
   (c) at least one alkanolamine, a C₁-C₄ alkyl alkanolamine, and mixtures thereof;
   (d) at least one oxidation dye, and
(ii) a second compartment comprises a hydrogen peroxide composition.

In another embodiment, the multi-compartment device for dyeing keratin fibers comprises at least two compartments packaged separate from one another, wherein
(i) one compartment comprises a tint composition, said tint composition comprising:
   (a) at least one hydrolyzed protein or peptide or amino acid, selected from the group consisting of hydrolyzed pea protein, a mixture comprising hydrolyzed soy protein, hydrolyzed wheat protein and hydrolyzed corn protein, and mixtures thereof;
   (b) hydrolyzed jojoba esters;
   (c) at least one alkanolamine or a C₁-C₄ alkyl alkanolamine, selected from the group consisting of monoethanolamine (MEA), diethanolamine, triethanolamine, isopropanolamine, 2-amino-1-propanol (2A1P), 2-amino-1-butanol (2A1B), 2-amino-2-methyl-1-propanol (AMP), 2-dimethylamino-2-methyl-1-propanol (DMAMP), tris(hydroxymethyl)aminomethane (tromethamine), N,N-diethylethanolamine (DEEA), N,N-dimethylethanolamine (DMEA), N-methyldiethanolamine (MDEA), N-methylethanolamine (NMEA), and mixtures thereof;
   (d) at least one oxidation dye, and
(ii) a second compartment comprises a hydrogen peroxide composition.

In another embodiment, the multi-compartment device for dyeing keratin fibers comprises at least two compartments packaged separate from one another, wherein
(i) one compartment comprises a tint composition, said tint composition comprising:
   (a) at least one hydrolyzed protein or peptide or amino acid, selected from the group consisting of hydrolyzed pea protein, hydrolyzed soy protein, hydrolyzed wheat protein, hydrolyzed corn protein, glycine, taurine, and mixtures thereof;
   (b) hydrolyzed jojoba esters;
   (c) at least one alkanolamine or a C₁-C₄ alkyl alkanolamine, selected from the group consisting of monoethanolamine (MEA), 2-amino-1-propanol (2A1P), 2-amino-1-butanol (2A1B), 2-amino-2-methyl-1-propanol (AMP), and mixtures thereof;
   (d) at least one oxidation dye, and
(ii) a second compartment comprises a hydrogen peroxide composition.

In another embodiment, the multi-compartment device for dyeing keratin fibers comprises at least two compartments packaged separate from one another, wherein
(i) one compartment comprises a tint composition, said tint composition comprising:
   (a) at least one hydrolyzed protein or peptide or amino acid, selected from the group consisting of hydrolyzed pea protein, a mixture comprising hydrolyzed soy protein, hydrolyzed wheat protein and hydrolyzed corn protein, and mixtures thereof;
   (b) hydrolyzed jojoba esters;
   (c) at least one alkanolamine or a C₁-C₄ alkyl alkanolamine, selected from the group consisting of monoethanolamine (MEA), 2-amino-1-propanol (2A1P), 2-amino-1-butanol (2A1B), 2-amino-2-methyl-1-propanol (AMP), and mixtures thereof;
   (d) at least one oxidation dye, and
(ii) a second compartment comprises a hydrogen peroxide composition.

In another embodiment, the multi-compartment device for dyeing keratin fibers comprises at least two compartments packaged separate from one another, wherein
(i) one compartment comprises a tint composition, said tint composition comprising:
   (a) at least one hydrolyzed protein or peptide or amino acid, selected from the group consisting of hydrolyzed pea protein, a mixture comprising hydrolyzed soy protein, hydrolyzed wheat protein and hydrolyzed corn protein, and mixtures thereof;
   (b) hydrolyzed jojoba esters;
   (c) monoethanolamine (MEA);
   (d) at least one oxidation dye, and
(ii) a second compartment comprises a hydrogen peroxide composition.

In another embodiment, the multi-compartment device for dyeing keratin fibers comprises at least two compartments packaged separate from one another, wherein
(i) one compartment comprises a tint composition, said tint composition comprising:
   (a) at least one hydrolyzed protein or peptide or amino acid, selected from the group consisting of a mixture comprising hydrolyzed soy protein, hydrolyzed wheat protein and hydrolyzed corn protein;
   (b) hydrolyzed jojoba esters;
   (c) monoethanolamine (MEA);
   (d) at least one oxidation dye, and
(ii) a second compartment comprises a hydrogen peroxide composition.

Typically, the multi-compartment device for dyeing keratin fibers is a multi-component packaging unit (kit-of-parts) including at least two compartments packaged separate from one another, wherein one compartment comprises a tint composition as defined above; and a second compartment comprises a hydrogen peroxide composition.

The tint composition as defined above and the hydrogen peroxide composition, packaged separate from one another, can be provided in separate containers, which are located together in an outer package (i.e. a cardboard box or a carton). Alternatively, the tint composition as defined above and the hydrogen peroxide composition can be provided in two separate containers, which are purchased separately from one another and are mixed together before use.

The tint composition as defined above and the hydrogen peroxide composition, packaged separate from one another, can also be provided in a single multi-chamber container (dispenser) such as an aerosol can.

When present, an optional conditioning agent can be provided in an additional container. In the latter case, the conditioner can be mixed immediately before use and applied together with the other components, or the content of the additional container can be applied (after an optional rinse step) as a post-treatment immediately after the composition for dyeing keratin fibers.

The following examples are given in order to provide a person skilled in the art with a sufficiently clear and complete explanation of the present disclosure, but should not be considered as limiting of the essential aspects of its subject, as set out in the preceding portions of this description.

### EXAMPLES

### 1. Preparation of the tint compositions

The compositions of Table 1 were prepared. CE1-CE3 represent Comparative Experiments.

**Table 1. - Tint compositions (wt. % based on the total weight of the composition).**

| **Ingredients (INCI)** | **C1** | **CE1** | **CE2** | **CE3** |
|---|---|---|---|---|
| Hydrolyzed Jojoba Esters (active matter) | 0.60 | --- | --- | 0.60 |
| Mixture of Hydrolyzed Wheat Protein, Hydrolyzed Soy Protein, Hydrolyzed Corn Protein (active matter) | 0.30 | --- | 0.30 | --- |
| Ceteareth-23 | 0.90 | 1.00 | 1.00 | 0.93 |
| Ceteareth-60 | 1.80 | 2.00 | 1.90 | 1.87 |
| Cetearyl alcohol | 3.60 | 4.00 | 3.90 | 3.72 |
| Cetyl alcohol | 1.60 | 1.80 | 1.70 | 1.68 |
| (Fatty Alcohols & Emulsifiers) | (7.9) | (8.8) | (8.5) | (8.2) |
| Behentrimonium Chloride | 0.80 | 0.80 | 0.80 | 0.80 |
| Monoethanolamine | 0.45 | 0.45 | 0.45 | 0.45 |
| Oxidation dyes to provide an Ash Light Blonde shade | 0.40 | 0.40 | 0.40 | 0.40 |
| Aqua (Water) (Eau), Propylene Glycol and Isopropyl Alcohol | 88.55 | 88.55 | 88.55 | 88.55 |
| Perfume, preservatives, antioxidants, reducing agents, chelating agents | Up to 100 | Up to 100 | Up to 100 | Up to 100 |

The compositions of Table 1 were prepared in such a way that the weight percentage of some of the ingredients remained constant in each composition such as Behentrimonium Chloride (a well-known hair conditioning agent); Monoethanolamine (alkanolamine); the oxidation dyes, the cosmetically acceptable medium (Water, Propylene Glycol and Isopropyl Alcohol), etc., but the weight percentage of the fatty alcohols Cetyl Alcohol and Cetearyl Alcohol and the emulsifiers (Ceteareth-23 and Ceteareth-60) was adjusted depending on whether Hydrolyzed Jojoba Esters and the hydrolized proteins were present or not. Thus, the reduction in the damage of the keratin fibers could only be attributed to the combination of hydrolized proteins and Hydrolyzed Jojoba Esters (Composition C1) and not to the fact that this composition had more "active" ingredients than Comparative Experiments CE1 to CE3.

### 2. Preparation of the dyeing compositions

Each tinting composition (C1, CE1, CE2 and CE3) were mixed in a non-metallic bowl at a ratio 1:2 by weight with a commercially available oxidizing composition, i.e. 3.2 wt.% (4 Vol) aqueous hydrogen peroxide composition (active matter to 1.2 wt.%) to obtain the dyeing compositions.

### 3. Application of the dyeing compositions

### 3.1. Pre-wash

Hair swatches of virgin (pure in nature and which has not been chemically processed or altered from its original state) dark Caucasian hair level 3 (according to the international system for measuring the shade of hair; shade scale ranging from 1-10 with 1 being the darkest shade and 10 being the lightest shade) from two different donors were selected, each one approximately 22 cm long and 1.5 g weight.

24 hours prior to the treatment, the swatches were washed with a shampoo and rinsed under tap water according to the following process:
- the swatches were soaked for 1 hour in a solution of 10 wt.% Pro You^{™} Neutral Shampoo (commercially available from Revlon Professional) and deonized water;
- the swatches were rinsed for 30 seconds under tap water;
- excess water was removed with a towel; and
- finally, the swatches were dried for 6 minutes using a Pro-Iron 3000 City hair dryer manufactured by Tectools and, after that, left to dry naturally.

### 3.2. Damaging (bleaching) hair

The hair swatches of step 3.1 were damaged according to the following process:
- the swatches were soaked for 1 hour at room temperature in a 2 wt.% hydrogen peroxide (active matter) and 0.77 wt.% ammonium hydroxide (active matter) aqueous solution;
- the swatches were soaked for 30 minutes at room temperature in a solution of 10 wt.% Pro You^{™} Neutral Shampoo (commercially available from Revlon Professional) and deonized water, under agitation (900 rpm);
- the swatches were rinsed for 30 seconds under tap water;
- excess water was removed with a towel; and
- finally, the swatches were dried for 6 minutes using a Pro-Iron 3000 City hair dryer manufactured by Tectools and, after that, left to dry naturally.

After the bleaching process, the color level of the hair swatches turned to chesnut (level 5).

Damage caused to the hair due to the bleaching process was measured using the method described below (point 4).

### 3.3. Application

The composition for dyeing keratin fibers C1 and the comparative experiments CE1-CE3 described above were applied to the bleached hair swatches of step 3.2.

The compositions were applied to the swatches for about 10 minutes and, after that, rinsed with an aqueous solution containing 150 ppm of water hardness ions (calcium and magnesium in a 3:1 weight ratio) and 1 ppm of cooper ions for about 5 minutes. Later,
- the hair swatches were soaked for 30 seconds at room temperature in a solution of 10 wt.% Pro You^{™} Neutral Shampoo (commercially available from Revlon Professional) and deonized water, under agitation (900 rpm);
- the swatches were rinsed for 30 seconds under tap water;
- excess water was removed with a towel.

This shampooing step was repeated 4 times to mimic the grooming routine between dyeing processes. After repeating the shampooing cycle 4 times, the hair swatches were dried for 6 minutes using a Pro-Iron 3000 City hair dryer manufactured by Tectools and, after that, left to dry naturally.

Damage caused to the hair due to the dyeing process was measured using the method described below (point 4).

### 4. Hair Damage Measurement via FT-IR

Damage caused to the hair due to the bleaching process (step 3.2. above) and due the dyeing process (step 3.3. above) was assessed by a FT-IR (Fourier Transform Infrared) method, as described by Signori, V. and Lewis, D.M., Int. J. Cosmet. Sci., 19, 1-13 (1997). Signori and Lewis (Int. J. Cosmet. Sci., 19, 1-13 (1997)) showed that FT-IR using a diamond Attenuated Total Internal Reflection (ATR) cell was a sensitive and reproducible way of measuring the cysteic acid content of single fibers and bundles.

A Perkin Elmer Spectrum Fourier Transform Infrared (FTIR) system equipped with a diamond Attenuated Total Internal Reflection (ATR) cell was used to measure the cysteic acid concentration of the hair surface. The swatches were platted (~1 plait per cm) in order to minimize variations in surface area of contact between readings. The ATR cell pressure was fixed at 70 points of the scale and remain the same pressure for all the swatches measurements.

The instrument was configured with a 4 cm⁻¹ resolution. The instrument was configured for performing 4 consecutive scans from 4000 to 450 cm⁻¹, getting as a result an average spectra of the 4 original scans. Five readings per swatch were taken (7 cm from the root of the swatch on both sides). Backgrounds were collected every 4 readings. The cell was cleaned with ethanol and a tissue between each reading. As prescribed by Signori and Lewis, a normalized double derivative analysis routine was used to get a quantitative result of the damage. The original spectra was initially converted to absorbance, before being normalized, where the intensity of the 1450 cm⁻¹ band (the characteristic and invariant protein CH2 stretch band) was fixed to an absorbance value of 0.00 and the intensity of the 1638 cm⁻¹ band (the characteristic and invariant Amide C=O stretch band) was fixed to an absorbance value of 1.50. This normalized absorbance spectra was then twice derivatized using a 37-point averaging. The intensity of the 1040 cm⁻¹ normalized 2nd derivative band was multiplied by -1×10⁻⁴ to recast it into suitable units.

The mean value of the five readings and the corresponding standard deviation per swatch was calculated.

The results are listed in Table 2 and Table 3. For each hair swatch, the hair damage measured after the dyeing process (step 3.3.) was normalized by the hair damage measured after the bleaching process (step 3.2).

**Table 2. - FT-IR Hair damage test results (cysteic acid units) - Donor 1**

| | C1 | | CE1 | | CE2 | | CE3 | |
|---|---|---|---|---|---|---|---|---|
| | Bleached Hair | Dyed Hair | Bleached Hair | Dyed Hair | Bleached Hair | Dyed Hair | Bleached Hair | Dyed Hair |
| Mean of five measurements | 20.484 | 14.568 | 19.822 | 16.380 | 17.718 | 17.134 | 18.448 | 16.630 |
| Standard deviation | 1.066 | 0.909 | 1.779 | 1.101 | 1.626 | 0.964 | 0.969 | 0.817 |
| Difference dyed hair - bleached hair | 5.916 | | 3.442 | | 0.584 | | 1.818 | |

**Table 3. - FT-IR Hair damage test results (cysteic acid units) - Donor 2**

| | C1 | | CE1 | | CE2 | | CE3 | |
|---|---|---|---|---|---|---|---|---|
| | Bleached Hair | Dyed Hair | Bleached Hair | Dyed Hair | Bleached Hair | Dyed Hair | Bleached Hair | Dyed Hair |
| Mean of five measurements | 22.314 | 14.364 | 14.544 | 22.054 | 16.624 | 14.846 | 19.236 | 15.346 |
| Standard deviation | 0.744 | 1.502 | 1.198 | 2.030 | 1.189 | 1.310 | 2.619 | 1.503 |
| Difference dyed hair - bleached hair | 7.950 | | - 7.510 | | 1.778 | | 3.890 | |

Minitab 17 Statistical Software was used to conduct a one-way Analysis of Variance (ANOVA) and Fisher individual test for differences of means of the results (significant level=0.05). The results are listed in Table 4 and Table 5.

**Table 4. - FT-IR Hair damage test results (cysteic acid units) - Mean values of Donor 1 and Donor 2**

| | C1 | CE1 | CE2 | CE3 |
|---|---|---|---|---|
| Difference dyed hair - bleached hair | 6.933 | - 2.034 | 1.181 | 2.854 |
| Standard deviation | 1.239 | 2.217 | 0.556 | 1.724 |

**Table 5. - Grouping Information using the Fisher LSD Method and 95% Confidence**

| Example | Mean | Statistical Group¹ | | |
|---|---|---|---|---|
| C1 | 6.933 | A | | |
| CE3 | 2.854 | | B | |
| CE2 | 1.181 | | B | |
| CE1 | - 2.034 | | | C |

| | | | | |
|---|---|---|---|---|
| ¹Means that do not share a letter are significantly different. | | | | |

The p-value was 0.000. Thus, less than the significance level of 0.05. Therefore, it can be concluded that there is a statistically significant difference between the mean obtained by Composition C1 and Comparative Experiments CE1, CE2 and CE3.

From the experimental results, it can be concluded that the dyeing compositions of the disclosure present reduced hair damage (less cysteic acid units) than the comparative experiments. In addition, the results show a synergistic effect in reducing the hair damage (cysteic acid units) for the composition C1 (composition comprising Hydrolyzed Proteins and Hydrolyzed Jojoba Esters) versus Comparative Experiments CE1, CE2 (composition comprising Hydrolyzed Proteins) and CE3 (composition comprising Hydrolyzed Jojoba Esters).

Modifications, which do not affect, alter, change or modify the essential aspects of the compositions and methods described above, are included within the scope of the present disclosure.

## Claims

1. A composition for dyeing keratin fibers comprising, in a cosmetically acceptable medium:
(a) at least one hydrolyzed protein or peptide or amino acid, and mixtures thereof,
(b) hydrolyzed jojoba esters;
(c) at least one alkanolamine, a C₁-C₄ alkyl alkanolamine, and mixtures thereof;
(d) at least one oxidation dye, and
(e) at least one oxidizing agent.

2. The dyeing composition according to claim 1, wherein the at least one hydrolyzed protein is selected from the group consisting of hydrolyzed silk protein, hydrolyzed pea protein, hydrolyzed soy protein, hydrolyzed wheat protein, hydrolyzed corn protein, and mixtures thereof.

3. The dyeing composition according to anyone of claims 1 to 2, wherein the at least one alkanolamine or C₁-C₄ alkyl alkanolamine is selected from the group consisting of monoethanolamine (MEA), 2-amino-1-propanol (2A1P), 2-amino-1-butanol (2A1B), 2-amino-2-methyl-1-propanol (AMP), 2-dimethylamino-2-methyl-1-propanol (DMAMP), tris(hydroxymethyl)aminomethane (tromethamine), N,N-diethylethanolamine (DEEA), N,N-dimethylethanolamine (DMEA), N-methyldiethanolamine (MDEA), N-methylethanolamine (NMEA), and mixtures thereof.

4. The dyeing composition according to claim 3, wherein the at least one alkanolamine or C₁-C₄ alkyl alkanolamine is selected from the group consisting of monoethanolamine (MEA), 2-amino-2-methyl-1-propanol (AMP), and mixtures thereof.

5. The dyeing composition according to anyone of claims 1 to 4, wherein the at least one oxidizing agent is hydrogen peroxide.

6. The dyeing composition according to anyone of claims 1 to 5, wherein the total amount of (a) the at least one hydrolyzed protein or peptide or amino acid, and mixtures thereof, is in the range of about 0.05 to 10 wt.%, based on the total weight of the composition.

7. The dyeing composition according to anyone of claims 1 to 6, wherein the total amount of (b) hydrolyzed jojoba esters is the range of about 0.05 to 10 wt.%, based on the total weight of the composition.

8. The dyeing composition according to anyone of claims 1 to 7, wherein the total amount of (c) the at least one alkanolamine, a C₁-C₄ alkyl alkanolamine, and mixtures thereof, is in the range of about 0.05 to 10 wt.%, based on the total weight of the composition.

9. The dyeing composition according to anyone of claims 1 to 8 comprising, in a cosmetically acceptable medium, based on the total weight of the composition:
(a) about 0.05-10 wt.% of at least one hydrolyzed protein or peptide or amino acid, and mixtures thereof,
(b) about 0.05-10 wt.% of hydrolyzed jojoba esters;
(c) about 0.05-10 wt.% of at least one alkanolamine, a C₁-C₄ alkyl alkanolamine, and mixtures thereof;
(d) about 0.001-10 wt.% of at least one oxidation dye, and
(e) about 0.05-10 wt.% of at least one oxidizing agent.

10. The dyeing composition according to claim 9, comprising, in a cosmetically acceptable medium, based on the total weight of the composition:
(a) about 0.05-10 wt.% of at least one hydrolyzed protein or peptide or amino acid, and mixtures thereof, selected from the group consisting of hydrolyzed silk protein, hydrolyzed pea protein, hydrolyzed soy protein, hydrolyzed wheat protein, hydrolyzed corn protein, and mixtures thereof;
(b) about 0.05-10 wt.% of hydrolyzed jojoba esters;
(c) about 0.05-10 wt.% of at least one alkanolamine, a C₁-C₄ alkyl alkanolamine, and mixtures thereof, selected from the group consisting of monoethanolamine (MEA), 2-amino-1-propanol (2A1P), 2-amino-1-butanol (2A1B), 2-amino-2-methyl-1-propanol (AMP), 2-dimethylamino-2-methyl-1-propanol (DMAMP), tris(hydroxymethyl)aminomethane (tromethamine), N,N-diethylethanolamine (DEEA), N,N-dimethylethanolamine (DMEA), N-methyldiethanolamine (MDEA), N-methylethanolamine (NMEA), and mixtures thereof;
(d) about 0.001-10 wt.% of at least one oxidation dye, and
(e) about 0.05-10 wt.% of at least one oxidizing agent.

11. The dyeing composition according to anyone of claims 1 to 10, wherein the keratin fibers are human hair.

12. The dyeing composition according to anyone of claims 1 to 12, wherein the dyeing composition has a pH in the range from 5.5 to 8.0.

13. A process for dyeing keratin fibers comprising the steps of:
i) applying a dyeing composition as defined in any of claims 1 to 12 to the keratin fibers for a period which is sufficient to obtain the dyeing effect;
ii) removing the dyeing composition from the keratin fibers by rinsing with water.

14. A process for dyeing keratin fibers according to claim 13, wherein the process is intended for reducing the damage of the keratin fibers.

15. A multi-compartment device for dyeing keratin fibers comprising at least two compartments packaged separate from one another, wherein
(i) one compartment comprises a composition for dyeing keratin fibers comprising:
(a) at least one hydrolyzed protein or peptide or amino acid, and mixtures thereof,
(b) hydrolyzed jojoba esters;
(c) at least one alkanolamine, a C₁-C₄ alkyl alkanolamine, and mixtures thereof;
(d) at least one oxidation dye, and
(ii) a second compartment comprises a hydrogen peroxide composition.
